# EUROPEAN PATENT SPECIFICATION

(11) **EP 2 957 571 B1**
(45) Date of publication and mention of the grant of the patent: **15.08.2018**
(21) Application number: 14305925.1
(22) Date of filing: 17.06.2014
(51) Int. Cl.: C07K 16/18, C07K 16/40

(54) **MONOCLONAL ANTI-PVHL ANTIBODIES AND USES THEREOF**
MONOKLONALE ANTI-PVHL-ANTIKÖRPER UND ANWENDUNGEN DAVON
ANTICORPS ANTI-PVHL MONOCLONAUX ET LEURS UTILISATIONS

(43) Date of publication of application: 23.12.2015
(73) Proprietor: CENTRE NATIONAL DE LA RECHERCHE SCIENTIFIQUE -CNRS-, 75794 Paris Cedex 16 (FR); Université de Rennes 1, 35000 Rennes (FR)
(72) Inventor: Arlot-Bonnemains, Yannick, 35235 THORIGNE FOUILLARD (FR); Chesnel, Franck, 35000 RENNES (FR); Le Goff, Xavier, 35310 Saint Thurial (FR); Hascoet, Pauline, 35170 BRUZ (FR); Couturier, Anne, 35160 LE VERGER (FR)
(74) Representative: Cabinet Plasseraud

(56) References cited:
- WO-A1-97/35978
- IMEN FERCHICHI ET AL: "Aurora A kinase interacts with and phosphorylates VHL protein", BIOLOGIA ; BOTANY, ZOOLOGY AND CELLULAR AND MOLECULAR BIOLOGY, SP VERSITA, HEIDELBERG, vol. 67, no. 5, 23 August 2012 (2012-08-23), pages 1026-1030, XP035101389, ISSN: 1336-9563, DOI: 10.2478/S11756-012-0091-0
- A BRADBURY ET AL: "Reproducibility: Standardize antibodies used in research.", NATURE, vol. 518, no. 7537, 5 February 2015 (2015-02-05), pages 27-29, XP055242695,

### SEQUENCE LISTING

<110> Universite de Rennes ARLOT-BONNEMAINS, Yannick
<120> Monoclonal Anti-pVHL Antibodies and Uses Thereof
<130> BEP130403EP
<160> 9
<170> PatentIn version 3.5
<210> 1
   <211> 213
   <212> PRT
   <213> Homo sapiens
<400> 1
   Met Pro Arg Arg Ala Glu Asn Trp Asp Glu Ala Glu Val Gly Ala Glu
   1               5                   10                  15
   Glu Ala Gly Val Glu Glu Tyr Gly Pro Glu Glu Asp Gly Gly Glu Glu
               20                  25                  30
   Ser Gly Ala Glu Glu Ser Gly Pro Glu Glu Ser Gly Pro Glu Glu Leu
           35                  40                  45
   Gly Ala Glu Glu Glu Met Glu Ala Gly Arg Pro Arg Pro Val Leu Arg
       50                  55                  60
   Ser Val Asn Ser Arg Glu Pro Ser Gln Val Ile Phe Cys Asn Arg Ser
   65                  70                  75                  80
   Pro Arg Val Val Leu Pro Val Trp Leu Asn Phe Asp Gly Glu Pro Gln
                   85                  90                  95
   Pro Tyr Pro Thr Leu Pro Pro Gly Thr Gly Arg Arg Ile His Ser Tyr
               100                 105                 110
   Arg Gly His Leu Trp Leu Phe Arg Asp Ala Gly Thr His Asp Gly Leu
           115                 120                 125
   Leu Val Asn Gln Thr Glu Leu Phe Val Pro Ser Leu Asn Val Asp Gly
       130                 135                 140
   Gln Pro Ile Phe Ala Asn Ile Thr Leu Pro Val Tyr Thr Leu Lys Glu
   145                 150                 155                 160
   Arg Cys Leu Gln Val Val Arg Ser Leu Val Lys Pro Glu Asn Tyr Arg
                   165                 170                 175
   Arg Leu Asp Ile Val Arg Ser Leu Tyr Glu Asp Leu Glu Asp His Pro
               180                 185                 190
   Asn Val Gln Lys Asp Leu Glu Arg Leu Thr Gln Glu Arg Ile Ala His
           195                 200                 205
   Gln Arg Met Gly Asp
       210
<210> 2
   <211> 160
   <212> PRT
   <213> Homo sapiens
<400> 2
   Met Glu Ala Gly Arg Pro Arg Pro Val Leu Arg Ser Val Asn Ser Arg
   1               5                   10                  15
   Glu Pro Ser Gln Val Ile Phe Cys Asn Arg Ser Pro Arg Val Val Leu
               20                  25                  30
   Pro Val Trp Leu Asn Phe Asp Gly Glu Pro Gln Pro Tyr Pro Thr Leu
           35                  40                  45
   Pro Pro Gly Thr Gly Arg Arg Ile His Ser Tyr Arg Gly His Leu Trp
       50                  55                  60
   Leu Phe Arg Asp Ala Gly Thr His Asp Gly Leu Leu Val Asn Gln Thr
   65                  70                  75                  80
   Glu Leu Phe Val Pro Ser Leu Asn Val Asp Gly Gln Pro Ile Phe Ala
                   85                  90                  95
   Asn Ile Thr Leu Pro Val Tyr Thr Leu Lys Glu Arg Cys Leu Gln Val
               100                 105                 110
   Val Arg Ser Leu Val Lys Pro Glu Asn Tyr Arg Arg Leu Asp Ile Val
           115                 120                 125
   Arg Ser Leu Tyr Glu Asp Leu Glu Asp His Pro Asn Val Gln Lys Asp
       130                 135                 140
   Leu Glu Arg Leu Thr Gln Glu Arg Ile Ala His Gln Arg Met Gly Asp
   145                 150                 155                 160
<210> 3
   <211> 172
   <212> PRT
   <213> Homo sapiens
<400> 3
<210> 4
   <211> 9
   <212> **PRT**
   <213> Homo sapiens
<400> 4
   Glu Ala Gly Arg Pro Arg Pro Val Leu
   1               5
<210> 5
   <211> 5
   <212> PRT
   <213> Homo sapiens
<400> 5
   Ala Gly Arg Pro Arg
   1               5
<210> 6
   <211> 19
   <212> DNA
   <213> Artificial Sequence
<220>
   <223> Forward Primer for VHL Variant 1
<400> 6
   cccgtatggc tcaacttcg 19
<210> 7
   <211> 22
   <212> DNA
   <213> Artificial Sequence
<220>
   <223> Reverse Primer for VHL variant 1
<400> 7
   tcaggtcgct ctacgaagat ct 22
<210> 8
   <211> 19
   <212> DNA
   <213> Artificial Sequence
<220>
   <223> Forward Primer for VHL Variant 2
<400> 8
   cccgtatggc tcaacttcg 19
<210> 9
   <211> 22
   <212> DNA
   <213> Artificial Sequence
<220>
   <223> Reverse Primer for VHL Variant 2
<400> 9
   tcaggtcgct ctacgaagat ct 22

## Description

### Background of the Invention

The World Health Organisation lists over 50 different types of kidney cancer. Renal Cell Carcinoma (RCC) is the most common type of kidney cancer in adults and amounts to up to 90% of all malignant kidney tumors. RCC is characterized by a lack of early warning signs, diverse clinical manifestations and resistance to chemotherapy and radiation. More than 50% of patients with early stage RCC are cured. Under certain circumstances, radical nephrectomy is also indicated to treat locally advanced RCC and metastatic disease after nephrectomy (Koul et al., Am. J. Cancer Res., 2011, 1(2): 240-254). However, the prognosis of advanced RCC is poor. Worldwide it is estimated that more than 200,000 new cases are diagnosed and more than 100,000 die from RCC each year. Both incidence and mortality of RCC are increasing worldwide. Age-specific incidence shows a peak in early childhood, then follows the more usual pattern of a steep rise through adulthood and the incidence of the disease is two to three times higher in men. Certain genetic conditions, such as von Hippel-Lindau (VHL) syndrome, are associated with an increased incidence of RCC.

The VHL syndrome is a rare, autosomal dominant genetic condition that predisposes individuals to highly vascularized benign and malignant tumors. The most common tumors found in VHL are renal cell carcinoma of the clear-cell type (ccRCC), hemangioblastoma (HB) and pheochromocytoma (tumor in the adrenal glands). Less frequent VHL tumors include those of the pancreas (pancreatic cysts, serous cystadenoma and pancreatic neuroendocrine tumors), inner ears (endolymphatic sac tumor) and testes (epididymal cystadenomas). The VHL syndrome results from a mutation in the *VHL* gene, and more than 70% of ccRCCs are characterized by somatic mutations or hypermethylation of VHL (Banks et al., Cancer Res., 2006, 66: 2000-2011; Herman et al., PNAS USA, 1994, 91: 9700-9704).

The *VHL* gene belongs to the family of tumor suppressor genes since biallelic mutations of the gene result in the development of malignant tumors. However, the molecular and cellular mechanisms by which this gene acts as a tumor suppressor are only poorly understood. Comparative studies using different model organisms have provided some insight into the numerous functions of pVHL, the protein encoded by the *VHL* gene (Hsu, Oncogene, 2012, 31: 2247-2257). The canonical function of pVHL is its role as the substrate-binding subunit of an E3 ubiquitin ligase. The best-known degradation target of VHL-containing E3 ligase is the α-subunit of hypoxia-inducible factor (HIF-α) in normal physiological conditions. Under normoxic conditions (*i.e.,* at normal oxygen level), HIF-α is hydroxylated at the proline residues within an oxygen-dependent domain. The prolyl-hydroxylated HIF-α is recognized by pVHL, leading to poly-ubiquitination and degradation. The hydroxylation reaction is mediated by the prolyl-hydroxylase domain proteins, which are only active under normoxic conditions. Under hypoxic conditions or when pVHL is defective, there is no interaction between pVHL and HIF-α. HIF-α, can then undergo dimerization with the β-submit (HIF-β) and the heterodimer formed translocates to the cell nucleus where it functions as a transcription factor, regulating genes encoding proteins involved in glycolysis (e.g., phosphoglycerate kinase), glucose transport (Glut-1), angiogenesis (VEGF) and erythropoiesis (erythroprotein), *i.e.,* proteins that mediate cellular response and adaptation to hypoxic conditions. pVHL has other functions that are non-canonical and independent from HIF-α, such as in fibronectin matrix assembly (Ohh et al., Mol. Cell., 1998, 1: 959-568) and in microtubules stability (Thoma et al., Nature Cell Biol., 2009, 11: 994-1001). However, in contrast to the canonical function of pVHL, these other functions are less known. The canonical function of pVHL alone cannot explain the formation of ccRCC tumors following mutation of the *VHL* gene. Indeed, studies have shown that HIF-α dysregulation is not sufficient to induce tumor formation in a mouse model (Elson et al., Genes Dev., 2001, 15: 2520-2532) and in humans (Percy et al., N. Engl. J. Med., 2008, 358: 162-168). It is therefore possible that the non-canonical functions reinforce the tumor suppressor role of pVHL. However, the link between the non-canonical functions of pVHL and cancer still remains to be established.

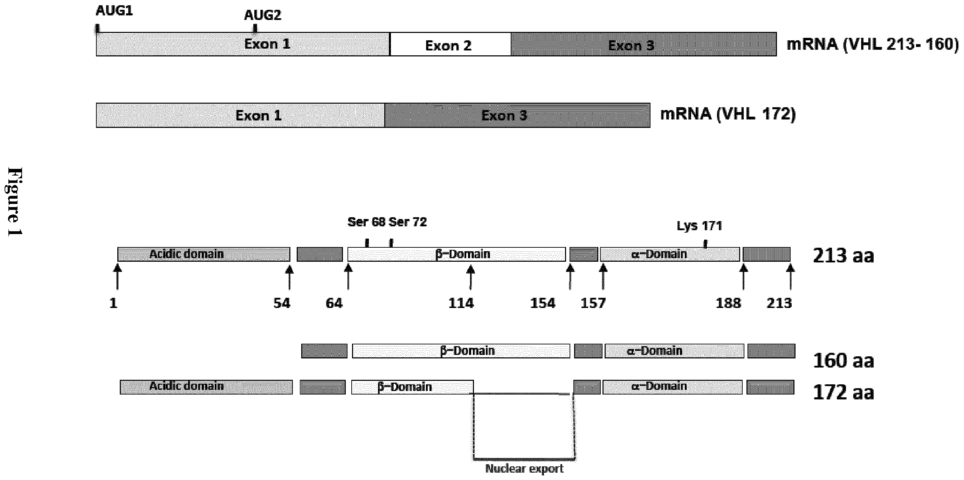

The complexity of the role of pVHL is not only due to its multifunctional aspect, but also to the fact that there are two different VHL mRNAs (messenger RNAs) encoding three isoforms of the VHL protein (Gnarra et al., Nature Genet., 1994, 7: 85-90; Richards et al., Human Mol. Genet., 1996, 5: 639-644). The first mRNA encodes isoforms pVHL213 and pVHL160 and the second mRNA encodes isoform pVHL172 (see Figure 1). pVHL213 is 213 amino acid-long and comprises an N-terminal acidic domain, a central β-domain, which is the binding site for HIF-α, and a C-terminal α-domain, which is the binding site for the members of the E3 ubiquitin-protein ligase complex (elongins B and C). pVHL160 is 160 amino acid-long and is identical to pVHL213 except that it does not comprise the N-terminal acidic domain. The third isoform, pVHL172, contains 172 amino acid residues and is identical to pVHL213 except that the C terminal part of the central β-domain is truncated. It has been shown that the isoforms differ in their subcellular localization, which may imply potential functional differences (Shraml et al., Am. J. Pathol., 2003, 163: 1013-1020; Hergovich et al., Nature Cell Biol., 2003, 5: 64-70). WO 97/35978, which relates to the VHL disease gene and its corresponding cDNA, mentions to the generation of antibodies to specific regions of human pVHL and Ferchichi et al. (Biologica, 2012, 67: 1026-1030) disclose the use of commercially available anti-pVHL antibodies. However, to this day, scientists have been unable to detect all three isoforms, and more specifically to detect pVHL172, and to study their respective roles in ccRCC tumorigenesis due to a lack of adequate tools.

Therefore, there still remains a need in the art for tools that allow for the three pVHL isoforms to be detected.

### Summary of the Invention

The present invention relates to tools, reagents and methods for detecting the three isoforms of the human pVHL protein, including the pVHL172 isoform. More specifically, the present Applicants have generated monoclonal antibodies that specifically recognize human pVHL172, pVHL213 and pVHL160. To the Applicants knowledge and based on experimental tests performed using commercially available anti-pVHL antibodies (data not shown), these are the first monoclonal antibodies capable of specifically binding to human pVHL172 (in addition to human pVHL213 and human pVHL160).

Accordingly, the present invention provides a hybridoma cell line deposited by the present Applicants at the CNCM (Collection Nationale de Cultures de Microorganismes, Institut Pasteur, 25 rue du Docteur Roux, 75724 Paris Cedex 15) on April 18, 2014 under Accession Number CNCM 1-4857.

The present invention also provides a monoclonal antibody secreted by the deposited hybridoma cell line, or a biologically active fragment thereof that specifically recognizes the three isoforms of human pVHL, *i.e.,* that specifically recognizes human pVHL172, in addition to human pVHL160 and pVHL213.

Preferably, the monoclonal antibody, or biologically active fragment thereof, specifically recognizes a region of human pVHL172 in the interdomain between the acidic domain and the β-domain of human pVHL172, wherein said region of human pVHL172 comprises, or consists of, the sequence set of forth in SEQ ID NO: 4 (EAGRPRPVL), or comprises, or consists of, the sequence set forth in SEQ ID NO: 5 (AGRPR).

In certain embodiments, the monoclonal antibody, or biologically active fragment thereof, is humanized, de-immunized or chimeric. For example, such a monoclonal antibody, or biologically active fragment thereof, may comprise the six complementary determining regions (CDRs) of the secreted monoclonal antibody described above.

The monoclonal antibody, or biologically active fragment thereof, may be immobilized on a solid support. For example, the solid support may be a bead, a particle, a microplate well, an array, a cuvette, a tube, a membrane, a gel, a resin, and the like.

The present invention also provides a conjugate comprising a monoclonal antibody, or a biologically active fragment thereof, according to the invention, which is attached (for example, which is covalently linked) to a detectable moiety.

In another aspect, the present invention provides a kit for detecting the presence of human pVHL in a biological sample or for quantifying the expression level of human pVHL in a biological sample or for isolating human pVHL from a biological sample, the kit comprising a monoclonal antibody, or a biologically active fragment thereof, according to the invention, or a conjugate according to the present invention.

In certain embodiments, the monoclonal antibody, or a biologically active fragment thereof, or the conjugate comprised in the kit is immobilized to a solid support.

The kit for detecting or quantifying human pVHL may be used to detect or quantify human pVHL172, human pVHL213, human pVHL160 or any combination thereof.

In certain embodiments, the kit further comprises a monoclonal antibody that specifically recognizes human pVHL213 and/or human pVHL160. Optionally, the monoclonal antibody that specifically recognizes human pVHL213 and/or human pVHL160 is attached (for example is covalently linked) to a detectable moiety. In certain embodiments, the kit further comprises an anti-human IgG optionally attached (for example is covalently linked) to a detectable moiety.

In yet another aspect, the present invention provides a method for detecting the presence of human pVHL in a biological sample or for quantifying the expression level of human pVHL in a biological sample, the method comprising a step of contacting the biological sample with a monoclonal antibody, or a biologically active fragment thereof, according to the invention, or with a conjugate according to the invention.

The method for detecting the presence of human pVHL in a biological sample or for quantifying the expression level of human pVHL may be used to detect or quantify human pVHL172, human pVHL213, human pVHL160 or any combination thereof.

In certain embodiments, the step of contacting the biological sample is performed for a time and under conditions allowing a complex to form between the human pVHL present in the biological sample and the monoclonal antibody, or biologically active fragment thereof, or the conjugate. In certain embodiments, the method further comprises a step of detecting any complex formed or quantifying any complex formed.

The method may be used for specifically detecting human pVHL213 and/or pVHL160 in the biological sample and/or of quantifying the expression level of human pVHL213 in the biological sample and/or the expression level of human pVHL160 in the biological sample.

The biological sample used in the method may be obtained from a patient, for example from a cancer patient (e.g., from a patient suffering from clear renal cell carcinoma or from another tumor associated with the VHL syndrome).

The present invention also provides a method for isolating human pVHL from a biological sample, the method comprising a step of contacting the biological sample with a monoclonal antibody, or a biologically active fragment thereof, according to the invention, or with a conjugate according to the invention.

The method for isolating human pVHL from a biological sample may be used to isolate human pVHL172, human pVHL213, human pVHL160 or any combination thereof from a biological sample.

In certain embodiments, the step of contacting the biological sample is performed for a time and under conditions allowing a complex to form between the human pVHL present in the biological sample and the monoclonal antibody, or biologically active fragment thereof, or the conjugate.

In certain embodiments, the monoclonal antibody, or biologically active fragment thereof, or the conjugate is immobilized to a solid support, and the method further comprises a step of releasing the pVHL bound to the monoclonal antibody, or biologically active fragment thereof, or the conjugate, which is immobilized to the solid support.

These and other objects, advantages and features of the present invention will become apparent to those of ordinary skill in the art having read the following detailed description of the preferred embodiments.

### Brief Description of the Drawing

**Figure 1****.** Schematic diagrams of pVHL protein: functional domains and amino acid sequence comparison of the three different pVHL isoforms.

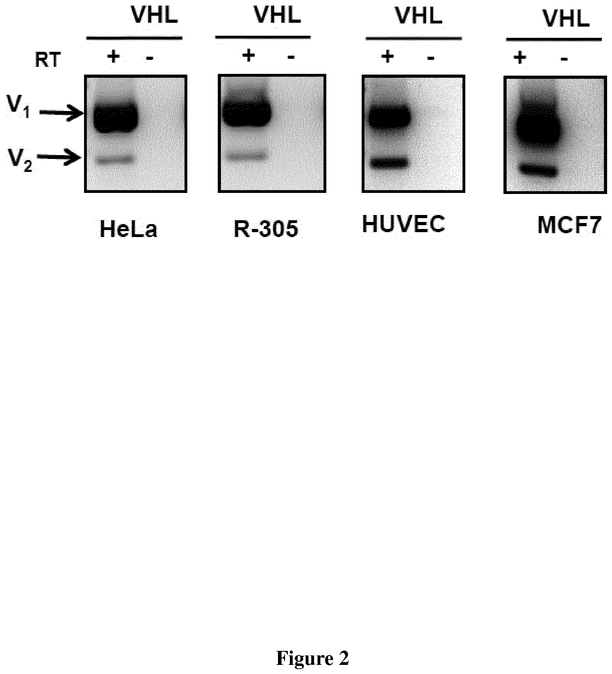

**Figure 2****.** Expression of VHL mRNA in R-305 HUVEC, HeLa, MCF-7 and cell lines. Total RNAs were extracted from cell extracts and reverse transcribed. The amount of VHL mRNA variants expression was analyzed by PCR using specific VHL primers producing an amplicon of 308 bp (VHL mRNA variant 1 (exon1-exon2-exon3)) and a second amplicon of 185 bp corresponding to VHL mRNA variant 2 (exon1-exon3).

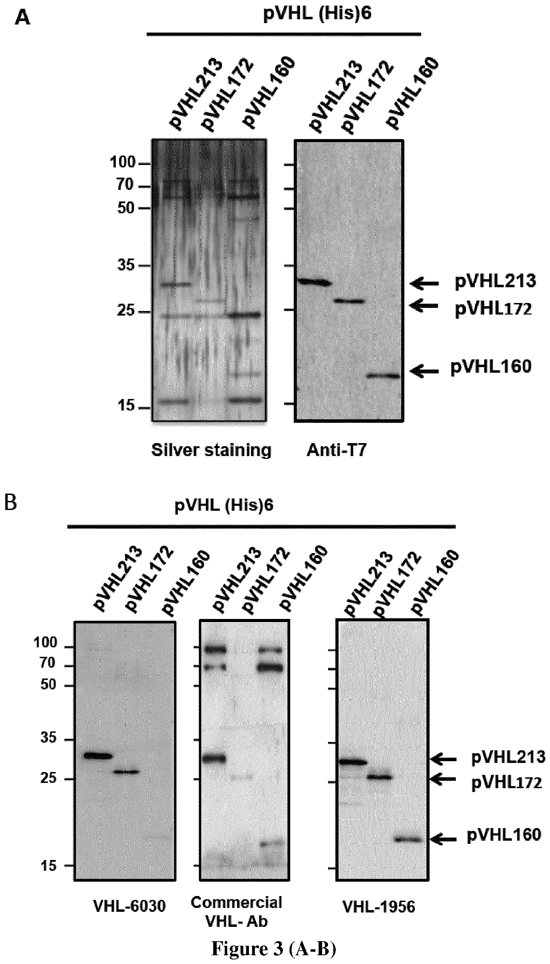

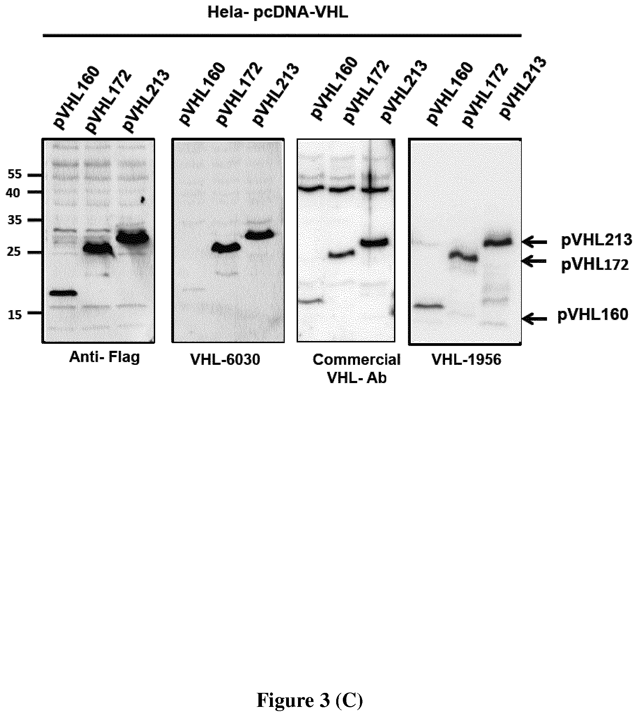

**Figure 3****.** Immunodetection of recombinant pVHL isoforms with different anti-pVHL antibodies **(A, B)** and immunodetection of pVHL isoforms overexpressed in HeLa cells **(C). (A)** Recombinant pVHL proteins were analyzed by Silver staining and by western-blotting using an anti-T7 antibody as a loading control. **(B)** The immunodetection for the recombinant proteins was performed with a polyclonal anti-VHL antibody: VHL-6030 (1:500), a commercial polyclonal anti-VHL antibody (Santa Cruz) (1:500) and the inventive monoclonal anti-VHL antibody: VHL-1956 (1:100). The immunoreactions were detected using the ELC kit detection from Pierce. **(C)** HeLa cells were transiently transfected with pcDNA plasmids encoding pVHL213, pVHL172 and pVHL160. Total proteins were analyzed by western-blotting using an anti-Flag antibody and a commercial polyclonal anti-VHL antibody (1:500), the VHL-6030 (1:500) and the inventive VHL-1956 (1:10) antibodies.

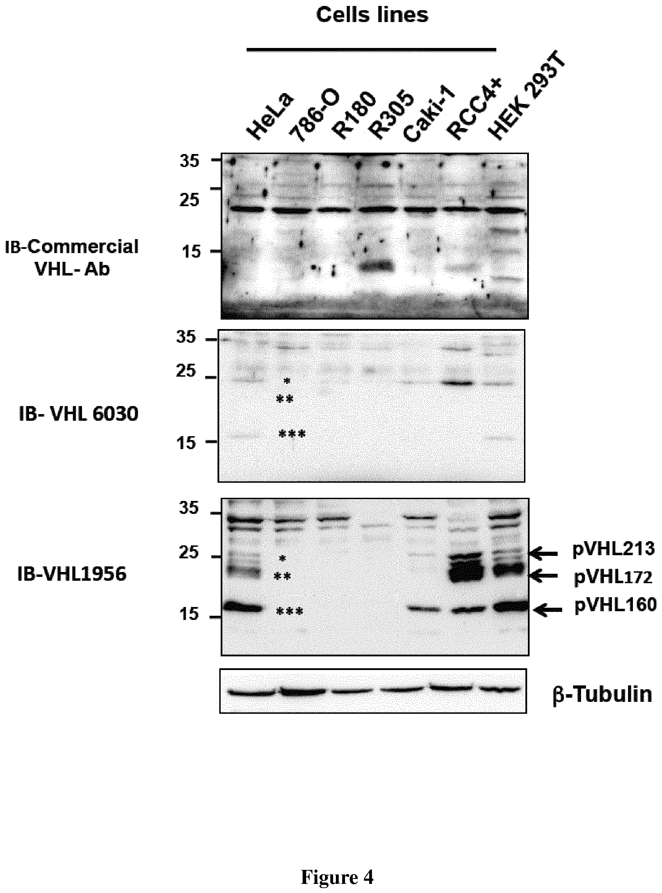

**Figure 4****.** Immunodetection of endogenous pVHL isoforms in cell lines. Forty (40) micrograms of total protein extracted from HeLa cells and kidney cells lines: HeLa, 786-O, R-180 and R-305 Caki-1, RCC4+ and HEK 293T cells were analyzed by western-blotting using three different anti-VHL antibodies: a commercial anti-VHL antibody (1:500), the antibody VHL-6030 (1:1500) and the inventive monoclonal antibody VHL-1956 (1:10). β-tubulin was used as a loading control. The immunoreaction was detected by chemiluminescence using the ECL kit from Pierce.

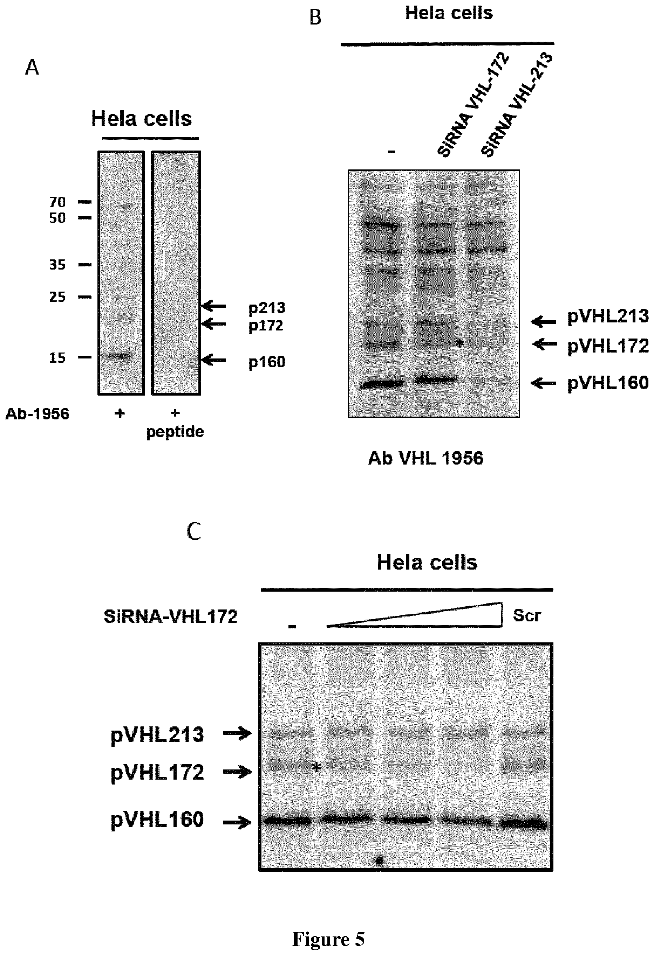

**Figure 5****.** Characterization of the inventive monoclonal VHL-1956 antibody.
   **(A)** Specificity of the antibody. Total protein extracts from HeLa cells were analyzed by western-blotting using the inventive monoclonal VHL-1956 antibody (left lane) or using the inventive monoclonal VHL-1956 antibody previously incubated for 30 minutes at 4°C with an excess of recombinant pVHL213 protein (right lane).
   **(B)** Knockdown of pVHL isoforms in HeLa cells. HeLa cells were transfected with siRNA (60 pmol) targeting all the three pVHL isoforms or only the pVHL172 isoform. Cells were harvested 24 hours after transfection and the amount of the different pVHL isoforms was assessed by western-blotting using VHL- and VHL-1956 (1:10) antibodies. Non-transfected HeLa cells were used to analyze the endogenous forms of pVHL isoforms. **(C)** HeLa cells were transfected using increasing amounts of siRNA targeting the pVHL172 isoform (20 pmol to 150 pmol) for 30 hours and then the expression of the pVHL isoforms was assessed by immunoblotting using the inventive VHL-1956 antibody (1:10). Non-transfected HeLa cells and HeLa cells transfected with scrambled siRNA (20 pmol) were used to analyze the expression of endogenous pVHL isoforms. All the immunoreactions were detected by chemiluminescence using the ECL kit from Pierce.

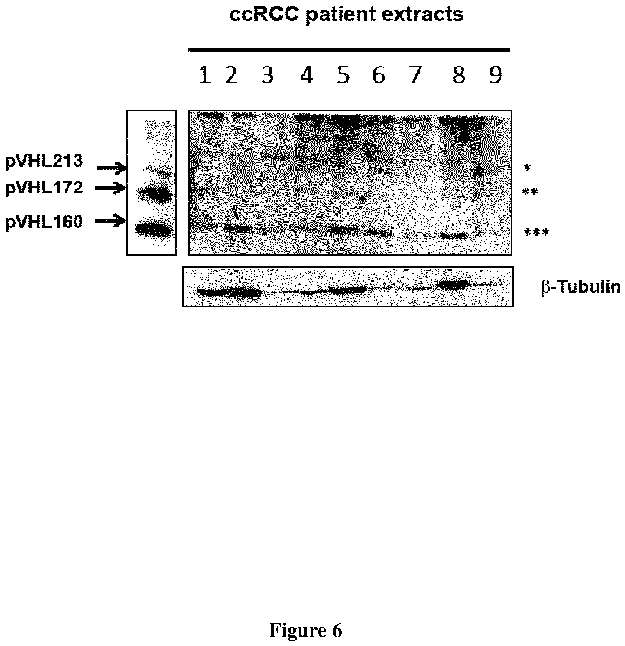

**Figure 6****.** Detection of pVHL using the inventive VHL-1956 antibody in tumor tissues. Western blot analysis of protein extracts from ccRCC tissue samples. Total proteins were extracted from ccRCC patient tissues using the RIPA buffer and analyzed by western-blotting using the VHL-1956 monoclonal antibody of the invention (1:10). β-tubulin was used as a loading control. Endogenous pVHL isoforms expression in HeLa cells was added in the left panel as a migration control of the three pVHL isoforms.

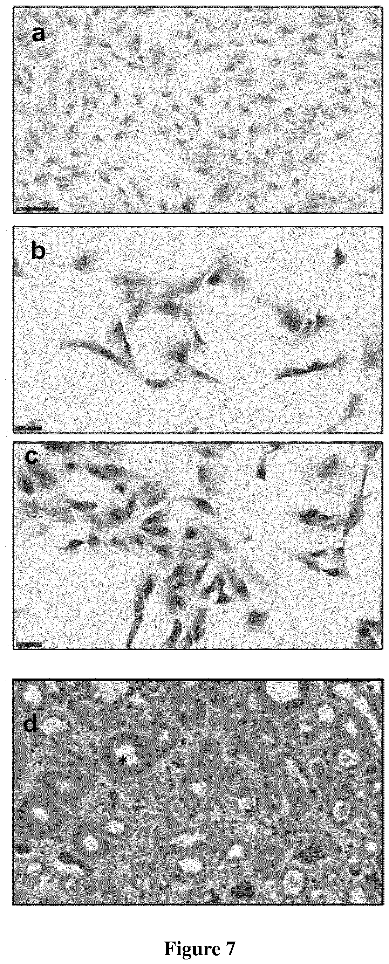

**Figure 7****.** The different cell lines 786-0 **(a),** 786-0 pVHL213 **(b)** and 786-0 pVHL172 **(c)** were cultivated on coverslips, fixed and processed for immunoreactive detection of pVHL in the presence of the antibody JD-1956 (dilution 1:50). Immunostraining was performed using the kit OmniMap DAB anti-rabbit (system "biotin-free") using technology multimer OmniMap anti-mouse HRPand ChromoMap DAB with antigen retrivial pH8. Control without primary antibody was always used at the same time, incubated with antibody dilutions. The same protocol was used with human kidney tissue. **(d)** Immunohistochemical analysis of VHL expression in ccRCC tissue samples with the VHL-1956 antibody of the invention. The tissue samples previously analyzed by western-blotting were prepared for immunohistochemistry and analyzed using the monoclonal VHL-1956 antibody. Microphotographs were acquired using a microscopie LEICA and the images were analyzed using the NDP view software.

### Definitions

Throughout the specification, several terms are employed that are defined in the following paragraphs.

The term *"**human pVHL"*** refers to the Von Hippel-Lindau tumor suppressor protein that is encoded by the *VHL* gene, which is located on the short arm of chromosome 3 (3p25-26). The terms *"**human pVHL213", "human pVHL160"*** and ***"human pVHL172**"* refer to the Von Hippel-Lindau tumor suppressor isoform 1, isoform 2 and isoform 3, respectively. More specifically, the terms ***"human pVHL213",*** and *"**human pVHL172"*** refer to proteins having, respectively, the sequence shown in GenBank Accession Number NP_000542.1 or any naturally occurring variant thereof, and the sequence shown in GenBank Accession Number NP_937799.1 or any naturally occurring variant thereof, respectively.

The term ***"antibody",*** as used herein, refers to immunoglobulin molecules and immunologically active portions of immunoglobulin molecules, *i.e.,* molecules that contain an antigen binding site that immunospecifically binds an antigen. As such, the term "antibody" encompasses not only whole antibody molecules, but also antibody fragments as well as variants (including derivatives) of antibodies and antibody fragments. A naturally occurring antibody is a glycoprotein comprising at least two heavy (H) chains and two light (L) chains inter-connected by disulphide bonds. There are two types of light chain, lambda (λ) and kappa (κ). There are five main heavy chain classes (or isotypes) which determine the functional activity of an antibody molecule: IgM, IgD, IgG, IgA and IgE. Heavy chains are classified as γ, µ, α, δ, or ε, which in turn define the immunoglobulin classes, IgG, IgM, IgA, IgD and IgE, respectively. Each heavy chain is comprised of a heavy chain variable region (V_{H}) and a heavy chain constant region (C_{H}). The heavy chain constant region is comprised of three domains, CH1, CH2 and CH3. Each light chain is comprised of a light variable region (V_{L}) and a light chain constant region (C_{L}). The V_{H} and V_{L} regions can be further subdivided into regions of hypervariability, termed complementary determining regions (CDR), interspersed with regions that are more conserved, termed framework regions (FR). Each V_{H} and V_{L} is composed of three CDRs and four FRs arranged from N-terminus to C-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component of the classical complement system. The term "antibody" encompasses monoclonal antibodies (which display a single binding specificity and affinity for a particular epitope) and polyclonal antibodies (*i.e.,* a collection of immunoglobulin molecules that react against a specific antigen, each identifying a different epitope). Antibodies according to the invention may be humanized antibodies or chimeric antibodies.

As used herein, the term *"**chimeric antibody**"* refers to an immunoglobulin molecule in which (a) the constant region, or a portion thereof, is altered, replaced or exchanged so that the antibody binding site (variable region) is linked to a constant region of a different or altered class, effector function and/or species, or an entirely different molecule which confers new properties to the chimeric antibody, *e.g.*, an enzyme, toxin, hormone, growth factor, drug, etc., or (b) the variable region, or a portion thereof, is altered, replaced or exchanged with a variable region, or portion thereof, having a different or altered antigen specificity, or with corresponding sequences from another species or from another antibody class or subclass.

As used herein, the term *"**humanized antibody**"* refers to an immunoglobulin molecule in which CDRs from a donor antibody are grafted onto human framework sequences. Humanized antibodies may also comprise residues of donor origin in the framework sequences. The humanized antibody can also comprise at least a portion of a human immunoglobulin constant region. Humanized antibodies may also comprise residues which are found neither in the recipient antibody nor in the imported CDR or framework sequences.

As used herein, the term ***"antibody fragment or derivative"*** refers to any derivative of an antibody which is less than full-length. In general, an antibody fragment retains at least a significant portion of the full-length antibody's specific binding ability. An antibody fragment may be produced by any means. While various antibody fragments are defined in terms of the enzymatic digestion of an intact antibody, one of skill will appreciate that fragments can be synthesized *de novo* either chemically or by utilizing recombinant DNA methodology. Thus, the term "antibody fragment or derivative", as used herein, includes antibody fragments either produced by the modification of whole antibodies or synthesized using other methodologies such as recombinant DNA methodologies. An antibody fragment or derivative may optionally comprise a single chain antibody fragment. Alternatively or additionally, an antibody fragment or derivative may comprise multiple chains which are linked together, for example, by disulfide linkages. An antibody fragment or derivative may optionally comprise a multimolecular complex. A functional antibody fragment or derivative typically comprises at least about 50 amino acids and more typically comprises at least about 200 amino acids. There are a number of well characterized antibody fragments or derivatives that retain specific binding including, but not limited to, Fab, Fab', F(ab')₂, scFv, Fv, dsFv diabody, and Fd fragments. Thus, for example, pepsin digests an antibody C-terminal to the disulfide linkages in the hinge region to produce F(ab')₂, a dimer of Fab which itself is a light chain joint to V_{H}-C_{H}1 by a disulfide bond. The F(ab')₂ may be reduced under mild conditions to break the disulfide linkage in the hinge region thereby converting the F(ab')₂ dimer into a Fab' monomer. The Fab' monomer is essentially a Fab with part of the hinge region. Antibody fragments include V_{H}-V_{L} dimers. A Fv fragment is the minimum antibody fragment which contains a complete antigen-recognition and binding site, it consists of one V_{H} domain and one V_{L} domain in tight, non-covalent association. A single chain Fv ("scFv") polypeptide is a covalently linked V_{H}-V_{L} heterodimer which is usually expressed from a gene fusion including V_{H}- and V_{L}-encoding genes linked by a peptide-encoding linker. A disulfide-stabilized Fv fragment ("dsFv") is a V_{H}-V_{L} heterodimer stabilized by a disulfide bond. Divalent and multivalent antibody fragments can form either spontaneously by association of monovalent scFvs, or can be generated by coupling monovalent scFvs by a peptide linker, such as divalent sc(Fv)₂. A Fd fragment consists of the V_{H} and C_{H}1 domains. A dAb fragment (Ward et al., Nature, 1989, 341: 544-546) is either the variable domain of an antibody heavy chain (V_{H} domain) or the variable domain of an antibody light chain (V_{L} domain). Each Dab thus contains 3 to 6 naturally occurring CDRs from an antibody. Antibody fragments can also be incorporated into single domain antibodies, maxibodies, minibodies, intrabodies, diabodies, triabodies, tetrabodies, v-NAR and bis-scFv (see, *e.g.,* Hollinger and Hudson, Nature Biotechnology, 2005, 23: 1126-1136). Antibody fragments can be incorporated into single chain molecules comprising a pair of tandem Fv segments (V_{H}-C_{H}1-V_{H}-C_{H}1) which, together with complementary light chain polypeptides, form a pair of antigen binding regions (Zapata et al., Protein Eng., 1995, 8: 1057-1062; U.S. Pat. No. 5,641,870).

An antibody, or antibody fragment or derivative thereof, is said to ***"recognize"*** a target polypeptide (or, more generally, a target molecule) if it finds and interacts with (e.g., binds to) the target polypeptide (or target molecule). Hence "recognition" involves the antibody binding reaction with the target polypeptide. The term ***"specifically*** (or selectively) ***recognize",*** when used in reference to an antibody, or an antibody fragment or derivative, refers to an antibody, or fragment or derivative, immunospecifically binding to a predetermined antigen. Typically, the antibody binds with an affinity of at least 1 x 10⁷ M⁻¹, and binds to the predetermined antigen with an affinity that is at least two-fold greater than the affinity for binding to a non-specific antigen (*e.g*., BSA, HSA, casein). In particular, an antibody or antibody fragment or derivative described herein specifically (or selectively) recognizes (*i.e.,* specifically or selectively binds to) human pVHL172. The terms *"**specifically or selectively recognizes the three isoforms of human pVHL"*** and ***"specifically or selectively binds to the three isoforms of human pVHL"*** are used herein interchangeably and refer to an antibody or antibody fragment or derivative that binds to human pVHL172, to human pVHL213 and to human pVHL160 but does not bind to any non-specific antigen.

The term ***"isolated",*** as used herein in reference to a protein or polypeptide (*e.g.,* a monoclonal antibody or a fragment or derivative thereof), means a protein or polypeptide, which by virtue of its origin or manipulation is separated from at least some of the components with which it is naturally associated or with which it is associated when initially obtained. By "isolated", it is alternatively or additionally meant that the protein or polypeptide of interest is produced or synthesized by the hand of man.

The terms ***"labeled", "attached to a detectable agent**"* and *"**attached to a detectable moiety"*** are used herein interchangeably. These terms are used to specify that an entity (*e.g.,* an antibody) can be visualized, for example, following binding to another entity (*e.g.,* an antigen). Preferably, a detectable agent or moiety is selected such that it generates a signal which can be measured and whose intensity is related to the amount of bound entity. Methods for labeling proteins and polypeptides, including antibodies, are well-known in the art. Labeled polypeptides can be prepared by incorporation of, or conjugation to, a label that is directly or indirectly detectable by spectroscopic, photochemical, biochemical, immunochemical, electrical, optical or chemical means, or any other suitable means. Suitable detectable agents include, but are not limited to, various ligands, radionuclides, fluorescent dyes, chemiluminescent agents, microparticles, enzymes, colorimetric labels, magnetic labels, and haptens.

The terms ***"protein", "polypeptide",*** and ***"peptide"*** are used herein interchangeably, and refer to amino acid sequences of a variety of lengths, either in their neutral (uncharged) forms or as salts, and either unmodified or modified by glycosylation, side-chain oxidation, or phosphorylation. In certain embodiments, the amino acid sequence is a full-length native protein. In other embodiments, the amino acid sequence is a smaller fragment of the full-length protein. In still other embodiments, the amino acid sequence is modified by additional substituents attached to the amino acid side chains, such as glycosyl units, lipids, or inorganic ions such as phosphates, as well as modifications relating to chemical conversions of the chains such as oxidation of sulfydryl groups. Thus, the term "protein" (or its equivalent terms) is intended to include the amino acid sequence of the full-length native protein, or a fragment thereof, subject to those modifications that do not significantly change its specific properties. In particular, the term "protein" encompasses protein isoforms, *i.e.,* variants that are encoded by the same gene, but that differ in their pI or MW, or both. Such isoforms can differ in their amino acid sequence (*e.g*., as a result of allelic variation, alternative splicing or limited proteolysis), or in the alternative, may arise from differential post-translational modification (*e.g*., glycosylation, acylation, phosphorylation). Proteins or polypeptides may be antibodies, antibody fragments, biologically active fragment thereof, and/or characteristic portions thereof.

The term ***"protein analog",*** as used herein in reference to a protein, refers to a polypeptide that possesses a similar or identical function as the protein but need not necessarily comprise an amino acid sequence that is similar or identical to the amino acid sequence of the protein or a structure that is similar or identical to that of the protein. Preferably, a protein analog has an amino acid sequence that is at least about 30%, more preferably, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95% or at least about 99% identical to the amino acid sequence of the protein.

The terms ***"fragment"*** and ***"portion",*** as used herein interchangeably in reference to a protein, refer to a polypeptide comprising an amino acid sequence of at least 5 consecutive amino acid residues (preferably, at least about: 10, 15, 20, 25, 30, 35, 40, 50, 60, 70, 80, 90, 100, 125, 150, 175, 200, 250 or more amino acid residues) of the amino acid sequence of a protein. The fragment of a protein may or may not possess a functional activity of the protein.

The term ***"biologically active",*** as used herein to characterize a protein (*e.g.,* an antibody) variant, a protein fragment or a protein derivative, refers to a molecule that shares sufficient amino acid sequence identity or homology with the protein to exhibit similar or identical properties to the protein. For, example, in many embodiments of the present invention, a biologically active fragment of an inventive monoclonal antibody is a fragment that retains the ability of the monoclonal antibody to recognize the three isoforms of human pVHL, including human pVHL172.

The term ***"homologous"*** (or ***"homology"***), as used herein, is synonymous with the term ***"identity"*** and refers to the sequence similarity between two polypeptide molecules or between two nucleic acid molecules. When a position in both compared sequences is occupied by the same base or same amino acid residue, the respective molecules are then homologous at that position. The percentage of homology between two sequences corresponds to the number of matching or homologous positions shared by the two sequences divided by the number of positions compared and multiplied by 100. Generally, a comparison is made when two sequences are aligned to give maximum homology. Homologous amino acid sequences share identical or similar amino acid sequences. Similar residues are conservative substitutions for, or "allowed point mutations" of, corresponding amino acid residues in a reference sequence. "Conservative substitutions" of a residue in a reference sequence are substitutions that are physically or functionally similar to the corresponding reference residue, *e.g.* that have a similar size, shape, electric charge, chemical properties, including the ability to form covalent or hydrogen bonds, or the like. Particularly preferred conservative substitutions are those fulfilling the criteria defined for an "accepted point mutation" as described by Dayhoff et al. ("Atlas of Protein Sequence and Structure", 1978, Nat. Biomed. Res. Foundation, Washington, DC, Suppl. 3, 22: 354-352).

As used herein, the term ***"subject"*** refers to a human or another mammal (*e.g*., primate, mouse, rat, rabbit, and the like). In many embodiments of the present invention, the subject is a human being. In such embodiments, the subject is often referred to as an ***"individual"*** or a ***"patient".*** The terms "individual" and "patient" do not denote any particular age. A "patient" is generally affected with a disease, disorder and/or medical condition (*e.g.*, has been diagnosed with a disease, disorder and/or medical condition, and/or displays one or more symptoms of the disease, disorder and/or medical condition).

The term ***"biological sample"*** is used herein in its broadest sense. A biological sample is generally obtained from a subject. A sample may be of any biological tissue or fluid that expresses the VHL protein. Frequently, a sample will be a "clinical sample", *i.e.,* a sample derived from a patient. Such samples include, but are not limited to, bodily fluids which may or may not contain cells, *e.g.,* blood (whole blood, serum or plasma), urine or saliva, tissue or fine needle biopsy samples, and archival samples with known diagnosis, treatment and/or outcome history. Biological samples may also include sections of tissues such as frozen sections taken for histological purposes. The term "biological sample" also encompasses any material derived from or obtained by processing the biological sample. Derived materials include, but are not limited to, cells (or their progeny) isolated from the sample, proteins or nucleic acid molecules extracted from the sample. Processing of a biological sample may involve one or more of: filtration, distillation, extraction, concentration, inactivation of interfering components, addition of reagents, and the like.

As used herein, the term ***"substantially"*** refers to the qualitative condition of exhibiting total or near-total extent or degree of a characteristic or property of interest. One of ordinary skill in the art will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

The terms ***"approximately"*** and ***"about",*** as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As mentioned above, the present invention provides monoclonal antibody molecules that specifically recognize human pVHL172 (along with the other two isoforms of pVHL, *i.e.* human pVHL213 and human pVHL1460), and a hybridoma cell line that secretes such monoclonal antibodies.

### I - Hybridomas and Anti-pVHL172 Antibodies

As shown in the Examples section below, the present Applicants have used genetic immunization of mice and screening methods to generate a hybridoma cell line which secretes monoclonal antibodies that specifically recognize the three isoforms of human pVHL, including human pVHL172. The terms *"anti-pVHL172 monoclonal antibody"* and *"anti-pVHL monoclonal antibody"* are used herein interchangeably and refer to a monoclonal antibody that binds to the three isoforms of human pVHL.

### A. Hybridoma Cell Line and Anti-pVHL172 Monoclonal Antibodies

Accordingly, the present invention provides a hybridoma cell line which secretes monoclonal antibodies that specifically recognize the three isoforms of human pVHL. More specifically, the monoclonal antibodies secreted by the hybridoma cell line of the present invention specifically recognize human pVHL172 as well as human pVHL213 and human pVHL160. In certain preferred embodiments, human pVHL213 has the sequence set forth in SEQ ID NO: 1 (GenBank Accession Number NP_000542.1) or a homologous sequence thereof resulting from genetic code degeneracy, human pVHL160 has the sequence set forth in SEQ ID NO: 2 or a homologous sequence thereof resulting from genetic code degeneracy, and human pVHL172 has the sequence set forth in SEQ ID NO: 3 (GenBank Accession Number NP_937799.1) or a homologous sequence thereof resulting from genetic code degeneracy, wherein:
SEQ ID NO: 1:
SEQ ID NO: 2:
SEQ ID NO: 3:

More specifically, the present invention provides a hybridoma cells line which secretes monoclonal antibodies that specifically recognize a region of human pVHL172 in the N-terminal portion of pVHL172. This region is common to human pVHL213 and human pVHL160. The region of human pVHL172 in the N-terminal portion of pVHL172 is in the interdomain between the acidic domain and the β-domain of pVHL172 (see Figure 1).

In particular, the hybridoma cell line of the present invention secretes monoclonal antibodies that specifically recognize a region of human pVHL172 in the interdomain between the acidic domain and the β-domain of pVHL172, wherein such region of human pVHL172 comprises, or consists of, the sequence set forth in SEQ ID NO: 4 (EAGRPRPVL). In particular, the hybridoma cell line of the present invention secretes monoclonal antibodies that specifically recognize a region of human VHL172 that consists of the sequence set forth in SEQ ID NO: 5 (AGRPR).

More specifically, the present invention provides a hybridoma cell line, which was generated by genetic immunization as described in the Examples section and which secretes monoclonal antibodies that specifically recognize the three isoforms of human pVHL, including human pVHL172. This hybridoma cell line, which is called JD1956 or VHL-1956, was deposited by the Applicants on April 18, 2014 at the CNCM (Collection Nationale de Cultures de Microorganisms, Institut Pasteur, 25 rue du Docteur Roux, 75724 Paris Cedex 15) under Accession Number CNCM 1-4857.

Also provided by the present invention are monoclonal antibodies secreted by this hybridoma cell line that specifically recognize pVHL172, pVHL213 and pVHL160, the three isoforms of human pVHL. Methods for the production and isolation of monoclonal antibodies from hybridoma cultures are well known in the art. Hybridoma cells are grown using standard methods, in suitable culture media such as, for example D-MEM and RPMI-1640 medium. An anti-pVHL monoclonal antibody can be recovered and purified from hybridoma cell cultures by protein A purification, ammonium sulphate or ethanol precipitation, acid extraction, anion or cation exchange chromatography, phosphocellulose chromatography, hydrophobic interaction chromatography, affinity chromatography, such as Protein A column, hydroxylapatite chromatography, lectin chromatography, or any suitable combination of these methods. High performance liquid chromatography (HPLC) can also be employed for purification.

Monoclonal antibodies of the present invention generally comprise any monoclonal antibody that is secreted by the hybridoma cell line of the invention (or a derivatized cell line) and that specifically recognizes the three isoforms of human pVHL, including human pVHL172, and in particular a region in the interdomain between the acidic domain and the β-domain of human pVHL172. The invention also encompasses any biologically active fragment of such a monoclonal antibody that retains the ability to specifically recognize the three isoforms of human pVHL, including human pVHL172, and in particular a region in the interdomain between the acidic domain and the β-domain of human pVHL172. In certain preferred embodiments, a monoclonal antibody according to the invention, or a biologically active fragment thereof, specifically recognizes a region of human pVHL172 that comprises, or consists of, the sequence set forth in SEQ ID NO: 4. In other preferred embodiments, a monoclonal antibody according to the invention, or a biologically active fragment thereof, specifically recognizes a region of human pVHL172 that comprises, or consists, of the sequence set forth in SEQ ID NO: 5.

Instead of using the hybridoma cell line described herein as a source for the antibodies, the monoclonal antibodies may be prepared by any other suitable method known in the art. For example, an inventive anti-pVHL monoclonal antibody may be prepared by recombinant DNA methods. These methods generally involve isolation of the gene encoding the desired antibody, transfer of the gene into a suitable vector, and bulk expression in a cell culture system. The gene or DNA encoding the desired monoclonal antibody may be readily isolated and sequenced using conventional procedures (*e.g.,* by using oligonucleotide probes that are capable of binding specifically to genes encoding the heavy and light chains of murine antibodies). The hybridoma cell line provided herein serves as a preferred source of such DNA. Suitable host cells for recombinant production of monoclonal antibodies include, but are not limited to, appropriate mammalian host cells, such as CHO, HeLa, or CV1. Suitable expression plasmids include, without limitation, pcDNA3.1 Zeo, pIND(SP1), pREP8 (all commercially available from Invitrogen, Carlsboad, CA, USA), and the like. The antibody genes may be expressed *via* viral or retroviral vectors, including MLV-based vectors, vaccinia virus-based vectors, and the like. Antibodies of the present invention may be expressed as single chain antibodies. Isolation and purification of recombinantly produced monoclonal antibodies may be performed as described above.

### B. Antibody Fragments

In certain embodiments, an inventive monoclonal antibody is used in its native form. In other embodiments, it may be truncated (*e.g., via* enzymatic cleavage or other suitable method) to provide immunoglobulin fragments or portions, in particular, fragments or portions that are biologically active. Biologically active fragments or portions of an inventive monoclonal antibody include fragments or portions that retain the ability of the monoclonal antibody to specifically recognize the three isoforms of human pVHL, including human pVHL172. In particular, biologically active fragments or portions of an inventive monoclonal antibody according to the invention retain the ability of the monoclonal antibody to specifically recognize a region in the interdomain between the acidic domain and the β-domain of human pVHL172. In particular, a biologically active fragment or portion of an inventive monoclonal antibody specifically recognizes a region of human pVHL172 that comprises, or consists of, the amino acid sequence set forth in SEQ ID NO: 4. In other preferred embodiments, a biologically active fragment or portion of an inventive monoclonal antibody specifically recognizes a region of human pVHL172 that comprises, or consists of, the amino acid sequence set forth in SEQ ID NO: 5. Biologically active fragments or portions of inventive monoclonal antibodies described herein are encompassed by the present invention.

A biologically active fragment or portion of an inventive monoclonal antibody may be an Fab fragment or portion, an F(ab')₂ fragment or portion, a variable domain, or one or more CDRs (complementary determining regions) of the antibody. Alternatively, a biologically active fragment or portion of an inventive monoclonal antibody may be derived from the carboxyl portion or terminus of the antibody protein and may comprise an Fc fragment, an Fd fragment or an Fv fragment.

Antibody fragments of the present invention may be produced by any suitable method known in the art including, but not limited to, enzymatic cleavage (e.g., proteolytic digestion of intact antibodies) or by synthetic or recombinant techniques. F(ab')₂, Fab, Fv and ScFv (single chain Fv) antibody fragments can, for example, be expressed in and secreted from mammalian host cells or from *E. coli.* Antibodies can also be produced in a variety of truncated forms using antibody genes in which one or more stop codons have been introduced upstream of the natural stop site. The various portions of antibodies can be joined together chemically by conventional techniques, or can be prepared as a contiguous protein using genetic engineering techniques.

### C. Fusion Proteins and Antibody Derivatives

Monoclonal antibodies (or fragments thereof), as described herein, may be produced in a modified form, such as a fusion protein (*i.e.,* an immunoglobulin molecule or portion linked to a polypeptide entity). Fusion proteins described herein retain the binding capability of the monoclonal antibody towards a region in the interdomain between the acidic domain and the β-domain of human pVHL172 (e.g., a region of human pVHL172 comprising, or consisting of, the sequence set forth in SEQ ID NO: 4 and/or a region of human pVHL172 comprising, or consisting of, the sequence set forth in SEQ ID NO: 5). A polypeptide entity to be fused to an inventive monoclonal antibody, or a fragment thereof, may be selected to confer any of a number of advantageous properties to the resulting fusion protein. For example, the polypeptide entity may be selected to provide increased expression of the recombinant fusion protein. Alternatively or additionally, the polypeptide entity may facilitate purification of the fusion protein, for example by acting as a ligand in affinity purification. A proteolytic cleavage site may be added to the recombinant protein so that the desired sequence can ultimately be separated from the polypeptide entity after purification. The polypeptide entity may also be selected to confer an improved stability to the fusion protein, when stability is a goal. Examples of suitable polypeptide entities include, but are not limited to, polyhistidine tags, that allow for the easy purification of the resulting fusion protein on a nickel chelating column. Glutathione-S-transferase (GST), maltose B binding protein, or protein A are other examples of suitable polypeptide entities.

Depending on the use intended, an antibody molecule described herein may be re-engineered so as to optimize stability, solubility, *in vivo* half-like, or ability to bind additional targets. Genetic engineering approaches as well as chemical modifications to accomplish any or all of these changes in properties are well known in the art. For example, the addition, removal, and/or modification of the constant regions of an antibody are known to play a particularly important role in the bioavailability, distribution, and half-life of administered antibodies. The antibody class and subclass, determined by the Fc or constant region of the antibody (which mediates effector functions), when present, imparts important additional properties. Thus, anti-pVHL antibodies are described that contain reconfigured, redesigned, or otherwise altered constant domains.

Additional fusion proteins may be generated through the techniques of DNA shuffling well known in the art (see, for example, U.S. Pat. Nos. 5,605,793; 5,811,238; 5,830,721; 5,834,252; and 5,837,458). DNA shuffling may be employed to modulate the activity of antibodies, or fragments thereof, for example, to obtain antibodies with higher affinity and lower dissociation rates. In such methods, polynucleotides encoding antibodies may be altered through random mutagenesis by error-prone PCR, random nucleotide insertion or other methods prior to recombination. Alternatively, one or more portions of a polynucleotide encoding an antibody described herein may be recombined with one or more components, motifs, sections, parts, domains, fragments, etc... of one or more heterologous molecules.

Alternatively, an antibody described herein may be linked to another antibody, *e.g.,* to produce a bispecific or a multispecific antibody. Methods for producing bispecific and multispecific antibodies are known in the art and include, for example, chemical synthesis involving cross-linking through reducible disulfide bonds or non-reducible thioether bonds, and recombinant methods.

### D. Chimeric/Humanized or De-immunized Antibodies

Anti-pVHL monoclonal antibodies of the present invention can also be "humanized": sequence differences between rodent antibodies and human sequences can be minimized by replacing residues which differ from those in the human sequences by site-directed mutagenesis of individual residues or by grafting of entire regions or by chemical synthesis. Humanized antibodies can also be produced using recombinant methods. In the humanized form of the antibody, some, most or all of the amino acids outside the CDR regions are replaced with amino acids from human immunoglobulin molecules, while some, most or all amino acids within one or more CDR regions are unchanged. Small additions, deletions, insertions, substitutions or modifications of amino acids are permissible as long as they do not abrogate the ability of the resulting antibody to specifically recognize the three isoforms of human pVHL, including human pVHL172, in particular a region in the interdomain between the acidic domain and the β-domain of human pVHL172 (*e.g.*, a region of human pVHL172 comprising, or consisting of, the sequence set forth in SEQ ID NO: 4 and/or a region of human pVHL172 comprising, or consisting of, the sequence set forth in SEQ ID NO: 5). Suitable human "replacement" immunoglobulin molecules include IgG1, IgG2, IgG2a, IgG2b, IgG3, IgG4, IgA, IgM, IgD or IgE molecules, and fragments thereof. Alternatively, the T-cell epitopes present in rodent antibodies can be modified by mutation (de-immunization) to generate non-immunogenic rodent antibodies that can be applied for therapeutic purposes in humans (see, for example, Accuro Biologics website).

Humanization can be performed using methods known in the art (*e.g.,* Jones et al., Nature, 1986, 321: 522-525; Riechmann et al., Nature, 1988, 332: 323-327; Verhoeyen et al., Science, 1988, 239: 1534-1536; Presta, Curr. Op. Struct. Biol., 1992, 2: 593-596; U.S. Pat. No. 4,816,567), including techniques such as "superhumanizing" antibodies (Tan et al., J. Immunol., 2002, 169: 1119) and "resurfacing" antibodies (*e.g.,* Staelens et al., Mol. Immunol., 2006, 43: 1243; and Roguska et al., Proc. Natl. Acad. Sci. USA, 1994, 91: 969).

### E. Antibody Conjugates

A monoclonal antibody of the invention, or a biologically active fragment thereof, may be functionally linked (*e.g.,* by chemical coupling, genetic fusion, non-covalent association or otherwise) to one or more other molecular entities. Methods for the preparation of such modified antibodies (or conjugated antibodies) are known in the art. (see, for example, "Affinity Techniques. Enzyme Purification: Part B", Methods in Enzymol., 1974, Vol. 34, W.B. Jakoby and M. Wilneck (Eds.), Academic Press: New York, NY; and M. Wilchek and E.A. Bayer, Anal. Biochem., 1988, 171: 1-32). Preferably, molecular entities are attached at positions on the antibody molecule that do not interfere with the recognition properties of the resulting conjugate, *i.e*., positions that do not participate in the specific binding of the antibody to human pHVL, and more particularly positions that do not participate in the specific binding of the antibody to a region in the interdomain between the acidic domain and the β-domain of human pVHL172 (e.g., a region of human pVHL172 comprising, or consisting of, the sequence set forth in SEQ ID NO: 4 and/or a region of human pVHL172 comprising, or consisting of, the sequence set forth in SEQ ID NO: 5).

In certain embodiments, the antibody molecule and molecular entity are directly covalently linked to each other. The direct covalent binding can be through a linkage such as a carbon-carbon, disulfide, amide, ester, carbamate, ether, thioether, urea, amino or carbonate linkage. Covalent binding can be achieved by taking advantage of functional groups present on the antibody and the molecular entity. An activating agent, such as a carbodiimide, can be used to form a direct linkage. In other embodiments, the antibody molecule and the molecular entity are covalently linked to each other through a linker group. This can be accomplished by using any of a wide variety of stable bifunctional agents well known in the art, including homofunctional and heterofunctional linkers.

An antibody of the present invention (or a biologically active fragment thereof) may be conjugated to a detectable agent or moiety. Any of a wide variety of detectable agents can be used in the practice of the present invention, including, without limitation, various ligands, radionuclides (*e.g*., ³H, ¹²⁵I, ¹³¹I, and the like), fluorescent dyes (*e.g*., fluorescein isothiocyanate, rhodamine, phycoerytherin, phycocyanin, allophycocyanin, o-phthalaldehyde and fluorescamine), chemiluminescent agents (*e.g.,* luciferin, luciferase and aequorin), microparticles (such as, for example, quantum dots, nanocrystals, phosphors and the like), enzymes (such as, for example, those used in an ELISA, *i.e.,* horseradish peroxidase, beta-galactosidase, luciferase, alkaline phosphatase), colorimetric labels, magnetic labels, and biotin, dioxigenin or other haptens and proteins for which antisera or monoclonal antibodies are available. The resulting detectable antibodies may be used in detection methods (see below).

Other molecular entities that can be conjugated to an antibody of the present invention (or a biologically active fragment thereof) include, but are not limited to, linear or branched hydrophilic polymeric groups, fatty acid groups, or fatty ester groups.

Thus, in addition to anti-pVHL monoclonal antibodies secreted by the hybridoma cell line described herein, and any biologically active fragments thereof, are also described herein chimeric antibodies, humanized antibodies, and antibody-derived molecules comprising at least one complementary determining region (CDR) from either a heavy chain or light chain variable region of a secreted anti-pVHL monoclonal antibody, including molecules such as Fab fragments, F(ab')₂ fragments, Fd fragments, Fabc fragments, Sc antibodies (single chain antibodies), diabodies, individual antibody light single chains, individual antibody heavy chains, chimeric fusions between antibody chains and other molecules, and antibody conjugates, such as antibodies conjugated to a diagnostic or therapeutic agent, which are all antibody derivatives. All these antibodies and antibody-related molecules described herein specifically recognize the three isoforms of human pVHL, including human pVHL172, in particular a region in the interdomain between the acidic domain and the β-domain of human pVHL172 (*e.g.,* a region of human pVHL172 comprising, or consisting of, the sequence set forth in SEQ ID NO: 4 and/or a region of human pVHL172 comprising, or consisting of, the sequence set forth in SEQ ID NO: 5).

### F. Activity and Specificity of Inventive Monoclonal Antibodies and Related Molecules

The antibodies, fragments and derivatives thereof may be assayed for specific binding by any method known in the art. Many different competitive binding assay formats can be used for specificity binding. The immunoassays which can be used include, but are not limited to, competitive assay systems using techniques such as Western blots, radioimmunoassays, ELISA, "sandwich" immunassays, immunoprecipitation assays, precipitin assays, gel diffusion precipitin assays, immunoradiometric assays, fluorescent immunoassays, protein A immunoassays, and complement-fixation assays. Such assays are routine and well known in the art (see, for example, Ausubel et al., eds, Current Protocols in Molecular Biology, 1994 , Vol. 1, John Wiley & sons, Inc., New York and "Antibodies, A Laboratory Manual", Cold Spring Harbor Laboratory, Ed Harlow and David Lane, 1988).

These methods can be used for testing supernatants from hybridomas producing antibodies, for testing the activity of isolated/purified monoclonal antibodies, and/or for testing the activity of modified antibodies (e.g., biologically active fragments thereof, derivatives thereof, conjugates thereof, etc). Binding specificity testing may be performed using the monoclonal antibody or monoclonal antibody-related molecule against a panel of cells, *e.g*., human cells, including kidney cell lines (such as, for example, Human Embryonic Kidney 293 cells or HEK 293 cells, human kidney carcinoma cell lines such as 769-P cells, 786-O cells or A-498 cells, human kidney adenocarcinoma cell lines such as A-407 cells or ACHN cells, human kidney clear cell carcinoma cell lines such as CaKi-1 or CaKi-2 cells, RCC-ER, RCC-GF1, RCC-FG2, RCC-GH, RCC-GS, RCC-HB; RCC-JF, RCC-JW, RCC-KL, RCC-KP, RCC-LR, RCC-MF, RCC-MH, RCC-OF1, RCC-PR or RCC-WK cells, human kidney cell lines such as RC-124 cells, or human kidney (Wilm's Tumor) cell lines such as SK-NEP1 cells or WT-CLS1 cells). Flow cytometry analysis can reveal binding specificity of the monoclonal antibody or monoclonal antibody-related molecule for the three isoforms of human pVHL, including human pVHL172, in various cell types.

### II - Uses of Anti-pVHL Monoclonal Antibodies

The antibodies (*i.e.,* anti-pVHL monoclonal antibodies excreted by the hybridoma cell line described herein) and modified antibodies (biologically active fragments or derivatives of anti-pVHL monoclonal antibodies excreted by the hybridoma cell line described herein and other antibody-related molecules, *e.g.*, chimeric antibodies, humanized antibodies, etc...) described herein may be employed as research tools and in a variety of applications, such as purification, and detection/diagnostic methods.

### A. Purification Methods

The monoclonal antibodies and antibody-related molecules described herein may be used as affinity purification agents. In this application, a monoclonal antibody or antibody-related molecule is immobilized on a solid phase such as Sephadex resin or filter paper, using methods well known in the art. The immobilized antibody is contacted with a sample containing human pVHL172 (or human pVHL213 or human pVHL160 or a combination thereof) to be purified, and thereafter the support is washed with a first suitable solvent that will remove substantially all the material in the sample except for the human pVHL172 protein (or human pVHL213 or human pVHL160 or combination thereof), which is bound to the immobilized antibody. Finally, the support is washed with another suitable solvent which will release the human pVHL172 protein (or human pVHL213 or human pVHL160 or a combination thereof) from the antibody. It is within the capability of one skilled in the art to select the first suitable solvent and second suitable solvent to achieve the desired isolation/purification of human pVHL172 (or human pVHL213 or human pVHL160 or a combination thereof) from the starting sample. Human pVHL172 (or human pVHL213 or human pVHL160 or a combination thereof) is thus isolated/purified from the sample.

### B. Detection/Diagnostic Methods

The monoclonal antibodies and antibody-related molecules described herein may be used in assays for detecting the presence of human pVHL (including human pVHL172) either *in vitro* or *in vivo* and/or for quantifying the expression level of human pVHL (including human pVHL172) in specific biological tissues, fluids or cells.

Detection assays described herein generally comprise contacting a biological sample with an inventive anti-pVHL monoclonal antibody (or a biologically active fragment thereof or derivative thereof) for a time and under conditions allowing a complex to form between the anti-pVHL monoclonal antibody (or fragment or derivative thereof) and human pVHL present in the biological sample; and detecting the presence or absence of the complex formed and/or determining the level of complex formed.

### Biological Samples

The methods may be applied to the study of any type of biological samples allowing human pVHL to be assayed. Examples of suitable biological samples include, but are not limited to, blood samples (*i.e.,* whole blood, serum or plasma), urine, saliva, synovial fluid, seminal fluid, lymphatic fluid, cerebrospinal fluid, peritoneal fluid, as well as endocervical, uretral, rectal, and vaginal samples. Biological samples may include sections of tissue (*e.g.*, kidney biopsy samples), frozen sections, and archival samples with known diagnosis, treatment and/or outcome history. Biological samples may also be cells (or their progeny) or cell content isolated from such tissues or fluids. Biological samples may be collected by any non-invasive means (*e.g.*, fine needle aspiration or biopsy) or obtained by surgical means (*e.g.,* nephrectomy).

The methods may be performed on the biological sample itself without, or with limited, processing of the sample.

However, alternatively, the methods may be performed on a protein extract prepared from the biological sample. In this case, the protein extract preferably contains the total protein content. Methods of protein extraction are well known in the art (see, for example, *"*Protein Methods", D.M. Bollag et al., 2nd Ed., 1996, Wiley-Liss; *"*Protein Purification Methods: A Practical Approach", E.L. Harris and S. Angal (Eds.), 1989; *"*Protein Purification Techniques: A Practical Approach", S. Roe, 2nd Ed., 2001, Oxford University Press; "Principles and Reactions of Protein Extraction, Purification, and Characterization", H. Ahmed, 2005, CRC Press: Boca Raton, FL). Various kits can be used to extract proteins from bodily fluids and tissues. Such kits are commercially available from, for example, BioRad Laboratories (Hercules, CA), BD Biosciences Clontech (Mountain View, CA), Chemicon International, Inc. (Temecula, CA), Calbiochem (San Diego, CA), Pierce Biotechnology (Rockford, IL), and Invitrogen Corp. (Carlsbad, CA). User Guides that describe in great detail the protocol to be followed are usually included in all these kits. Sensitivity, processing time and costs may be different from one kit to another. One of ordinary skill in the art can easily select the kit(s) most appropriate for a particular situation.

### Detection of Antibody-pVHL Complexes

The methods described herein generally involve detecting at least one complex formed between the anti-pVHL monoclonal antibody (or fragment or derivative thereof) added to the biological sample and human pVHL (including human pVHL172) present in the biological sample. Detection of such a complex may be performed by any suitable method known in the art (see, for example, E. Harlow and A. Lane, "Antibodies: A Laboratories Manual", 1988, Cold Spring Harbor Laboratory: Cold Spring Harbor, NY).

For example, detection of an antibody-pVHL complex may be performed using an immunoassay. A wide range of immunoassay techniques can be used, including radioimmunoassays, enzyme immunoassays (EIA), enzyme-linked immunosorbent assays (ELISA), and immunofluorescence immunoprecipitation. Immunoassays are well known in the art. Methods for carrying out such assays as well as practical applications and procedures are summarized in textbooks. Examples of such textbooks include P. Tijssen, In: Practice and theory of enzyme immunoassays, eds. R.H. Burdon and v. P.H. Knippenberg, Elsevier, Amsterdam (1990), pp. 221-278 and various volumes of Methods in Enzymology, Eds. S.P. Colowick et al., Academic Press, dealing with immunological detection methods, especially volumes 70, 73, 74, 84, 92 and 121. Immunoassays may be competitive or non-competitive.

For example, any of a number of variations of the sandwich assay technique may be used to perform an immunoassay. Briefly, in a typical sandwich assay applied to the detection of human pVHL according to the present invention, an unlabeled anti-pVHL monoclonal antibody (or biologically active fragment thereof) is immobilized on a solid substrate and the sample to be tested is brought into contact with the bound antibody for a time and under conditions allowing formation of an antibody-pVHL complex. Following incubation, a secondary antibody that is labeled with a detectable moiety and that specifically recognizes human antibodies (*e.g*., an anti-human IgG) is added and incubated under conditions allowing the formation of a ternary complex between any antibody-pVHL complex formed and the labeled secondary antibody. Any unbound material is washed away, and the presence of any pVHL in the biological sample is determined by observing the signal directly or indirectly produced by the detectable moiety. Variations in this assay include an assay in which both the biological sample and the labeled secondary antibody are added simultaneously to the immobilized anti-pVHL monoclonal antibody (or biologically active fragment thereof).

The secondary antibody may be labeled with any suitable detectable moiety, *i.e.,* any entity which, by its chemical nature, provides an analytical identifiable signal allowing detection of the ternary complex, and consequently detection of the antibody-pVHL complex.

The monoclonal antibody (or biologically active fragment or derivative thereof) may be immobilized by being either covalently or non-covalently bound to the surface of a solid carrier or support. The solid support may be any solid support known in the art to which the antibody can be operably affixed. "Operably affixed" refers to the antibody being affixed in a manner permitting the formation of a complex between the affixed antibody and human pVHL present in the biological sample tested. Examples of suitable carrier or support materials include, but are not limited to, agarose, cellulose, nitrocellulose, dextran, Sephadex, Sepharose, carboxymethyl cellulose, polyacrylamides, polystyrene, polyvinyl chloride, polypropylene, gabbros, magnetic ion-exchange resin, glass, polyamine-methyl-vinyl-ether-maleic acid copolymer, amino acid copolymer, ethylene-maleic acid copolymer, nylon, silk, and the like. Immobilization of an antibody on the surface of a solid carrier or support may involve crosslinking, covalent binding or physical adsorption, using methods well known in the art. Alternatively, the solid carrier or support may be in the form of a bead, a particle, a microplate well, an array, a cuvette, a tube, a membrane or any other shape suitable for condition an immunoassay. Immobilization of an antibody (or biologically active fragment or derivative thereof) to a solid carrier or support includes gel electrophoresis followed by transfer to a membrane (typically nitrocellulose or PVDF) in a process called Western blotting (or immunoblot) well known in the art.

Detection of the complex formed may be either qualitative or quantitative. Methods for labeling biological molecules such as antibodies are well-known in the art (see, for example, "Affinity Techniques. Enzyme Purification: Part B", Methods in Enzymol., 1974, Vol. 34, W.B. Jakoby and M. Wilneck (Eds.), Academic Press: New York, NY; and M. Wilchek and E.A. Bayer, Anal. Biochem., 1988, 171: 1-32).

The most commonly used detectable moieties in immunoassays are enzymes and fluorophores. In the case of an enzyme immunoassay (EIA), an enzyme such as horseradish peroxidase, glucose oxidase, beta-galactosidase, alkaline phosphatase, and the like, is conjugated to the second antibody, generally by means of glutaraldehyde or periodate. The substrate to be used with the specific enzymes is generally chosen for the production of a detectable color change, upon hydrolysis of the corresponding enzyme. In the case of immunofluorescence, the second antibody is chemically coupled to a fluorescent moiety without alteration of its binding capacity. After binding of the fluorescently labeled antibody to the antibody-pVHL complex and removal of any unbound material, the fluorescent signal generated by the fluorescent moiety is detected, and optionally quantified. Alternatively, the second antibody may be labeled with a radioisotope, a chemiluminescent moiety, or a bioluminescent moiety.

In certain embodiments, the presence of human pVHL or quantity thereof in a biological sample obtained from a patient may be used as an indication of the presence of a given condition (*e.g.,* kidney cancer, such as ccRCC, hemangioblastoma or pheochromacytoma or other tumors associated with the VHL syndrome). In certain methods, the quantity of antibody-pVHL complex measured in the biological sample tested is compared to the quantity of antibody-pVHL complex formed under the same conditions in a biological sample obtained from a healthy subject (or from a series of biological samples obtained from a significant number of healthy subjects).

### III - Kits

In another aspect, the present invention provides kits comprising materials useful for carrying out the separation methods or the detection/quantification/diagnostic methods of the invention. The methods described herein may be performed by diagnostic or analysis laboratories, research laboratories or practitioners. The invention provides kits that can be used in these different settings.

Materials and reagents for detecting or quantifying human pVHL (including human pVHL172) in biological samples or for isolating/separating human pVHL from biological samples according to a method of the invention may be assembled together in a kit. An inventive kit generally comprises a monoclonal anti-pVHL antibody of the invention, or a biologically active fragment thereof. The amount of monoclonal anti-pVHL antibody, or biologically active fragment thereof, in a kit may be varied to allow the separation, detection and/or quantification of human pVHL in a given number of biological samples, for example, one biological sample, or more than one biological sample, for example 2, 5, 10, 25, 50, 75, 100 or more biological samples. The monoclonal anti-pVHL antibody, or biologically active fragment thereof, may or may not be immobilized on a substrate surface (*e.g.*, beads, arrays, sorbents, resins, and the like).

In certain embodiments, a kit according to the present invention further comprises a monoclonal antibody that specifically recognizes human pVHL213 and/or a monoclonal antibody that specifically recognizes human pVHL160. To the Applicants knowledge, antibodies currently commercially available recognize in a non-specific manner pVHL213 and/or pVHL160. These antibodies are commercialized, for example, by Lifespan Biosciences, Thermo Fisher Scientific, Pierce Antibodies, Epitomics Abcam, BD Pharmingen, Sigma, Novus Biological and Origene.

In certain embodiments, a kit according to the present invention further comprises an anti-human IgG, which may or may not be labelled with a detectable moiety.

Depending on the procedure and/or the nature of the biological sample, the kit may further comprise one or more of extraction buffer and/or reagents, fixing buffer and/or reagents, lysis buffer and/or reagents, separation buffer and/or reagents, eluting solvent and/or reagents, labeling buffer and/or reagents, etc.... Protocols for using these buffers and reagents for performing different steps of the procedure may be included in the kit.

A kit of the invention may further comprise isolated, pure human pVHL172, pure human pVHL213, and/or pure human pVHL160 as a standard for establishing a standard curve.

The monoclonal anti-pVHL antibody, or biologically active fragment thereof, and all the other reagents may be supplied in a solid (*e.g*., lyophilized) or liquid form. The kits of the present invention may comprise different containers (*e.g.*, vials, ampoules, test tubes, flasks or bottles) for each individual buffer and/or reagent. Each component will generally be suitable as aliquoted in its respective container or provided in a concentrated form. Other containers suitable for conducting certain steps of the disclosed methods may also be provided. The individual containers of the kit are preferably maintained in close confinement for commercial sale.

A kit may further comprise instructions for using the kit according to a method of the invention. Such instructions may comprise instructions for processing the biological sample obtained and/or for performing the test, instructions for interpreting the results as well as a notice in the form prescribed by a governmental agency (*e.g.,* the FDA and/or the European Medecines Agency) regulating the manufacture, use or sale of pharmaceuticals or biological products.

An identifier, *e.g.,* a bar code, radio frequency, ID tags, etc., may be present in or on the kit packaging. The identifier can be used, for example, to uniquely identify the kit for purposes of quality control, inventory control, tracking movement between workstations, etc.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or data are actually obtained.

### Materials and Methods

**Real Time PCR Analysis.** Real time PCR was performed on total RNA extracted from tumor samples using the Qiagen Qiamp total RNA kit, as follows: 5 micrograms of total RNA were reverse-transcribed using oligo-dT primers and M-MLV reverse transcriptase. The resulting cDNAs were then used as templates for the subsequent PCR amplifications of Aurora-A and GAPDH used as internal controls. Primers designed from human cDNA sequences were: 5'-CCCGTATGGCTCAACTTCG-3' (SEQ ID NO: 6) (forward) and 5'-TCAGGTCGCTCTACGAAGATCT-3' (reverse) (SEQ ID NO: 7) for VHL variant 1 (308bp), 5' CCCGTATGGCTCAACTTCG-3' (forward) (SEQ ID NO: 8) and 5'-TCAGGTCGCTCTACGAAGATCT-3' (reverse) (SEQ ID NO: 9) for VHL variant 2 (185 bp). Assays were performed in triplicate, using the RotorGene 3000 instrument (Corbett Research, Biolabo, Archamps, France) with SYBR Green I master mix (Roche Diagnostics, Mannheim, Germany). To avoid the formation of primer-dimer artefacts, fluorescence was monitored at a temperature above the melting point of primer-dimer but below the melting point of the specific PCR product. For each sample, the relative amounts of VHL and GAPDH transcripts were calculated from these standard curves using the RotorGene software.

**Protein Purification.** All recombinant proteins were prepared from *E. coli* strain BL21(DE3)pLysS induced to overexpress the proteins VHL213(His)₆, VHL172(His)₆ and VHL160 (His)₆. The bacteria were lyzed in the IMAC 5 buffer (20 mM Tris-Hcl pH 7.5; 500 mM NaCl; 10% glycerin and 5 mM imidazol) with 1 mg/ml lysozyme and 1 mM PMSF for 1 hour at 4°C. The bacteria lysates were then centrifuged for 30 minutes at 12,000g at 4°C (JA-20 rotor, Beckman) and the supernatants were filtered. The proteins were then purified by Ni-NTA-agarose affinity chromatography following the manufacturer's instructions (Qiagen SA). The beads were washed twice with 10 volumes of IMAC 5 and were then incubated in 5% BSA in IMAC 5 for 1 hour at 4°C. The supernatants were incubated with the beads for 3 hours at 4°C and the beads were washed twice with 10 volumes of IMAC 5 and three times with 10 volumes of IMAC20 (20 mM Tris-HCl pH 7.5; 500 mM NaCl; 10% glycerol; 20 mM imidazole). The His-tagged proteins were eluted with IMAC 250 (IMAC-250 mM imidazol). The eluted fractions were controlled in concentration by Bradford analysis and for purity by electrophoresis onto a 12.5% SDS gel (Bradford, Anal. Biochem., 1976, 72: 248-254).

**Antibodies.** The polyclonal antibodies against VHL were obtained in the laboratory (Martin et al., PLOs One, 2013, 8(6)e67071), the actin antibody was purchased from Sigma (USA). The monoclonal antibody against VHL was produced in the Applicants' laboratory (CNRS-EFS). The commercial antibody against VHL was from Santa Cruz. The horseradish peroxidase-conjugated secondary antibodies were from Jackson Immuno-Research Laboratories (Baltimore, MD).

**ELISA Technique.** Fifty microliters of VHL213, VHL172 or irrelevant protein (2 µg/ml) were diluted in carbonate buffer (50 mM, pH 9.6) and coated in a 96-well ELISA plate (Medisorp, Nunc). The plates were incubated at 2-8°C overnight. After washing three times with PBST (10 mM of phosphate buffer pH 7.2, 0.05 % Tween-20) the blockage of non-specific binding sites was realized with PBST-5% bovine serum albumin. Following incubation of 1 hour to 2 hours at room temperature, the plates were flipped and 50 µL of hybridoma cell supernatant were then added to each well. The plate was further incubated for 1 to 3 hours at 37°C and washed 3 times with PBST. Fifty microliters of a horseradish peroxidase conjugated goat anti-mouse IgG (Sigma) diluted 1/5 000 in PBS-0.1% BSA-0.01% Tween20 were added and the plate was further incubated for 1 hour at 37°C. Revelation was carried out after 3 washes and addition of 50 µL of substrate solution per well (1 mg/ml) of ABTS in 0.05 M phosphate-citrate buffer, pH 5.0. The absorbance was measured at 405 nm after 10 to 30 minutes incubation using a Thermo Scientific Multiskan EX photometric microplate absorbance reader with Ascent software v2.6 (Thermo Labsystems).

**Western Blot Analysis.** Protein extract frozen tissues were suspended in RIPA buffer (50 mM Tris-HCl, pH 7.4, 1% NP-40, 0.5% sodium deoxycholate, 150 mM sodium chloride, 1 mM EDTA, 1 mM sodium fluoride, 1 mM AEBSF, 10 µg/ml aprotinin, 10 µg/ml leupeptin, 1 mM sodium orthovanadate), and centrifuged at 9000g for 10 minutes. Bradford assays were performed to determine protein concentrations in supernatants. Fifty µg of total proteins from each extract were electrophoresed on a 15% polyacrylamide gel and transferred onto nitrocellulose membranes. The membranes washed with TBST were saturated with 5% low fat milk in TBST for 2 hours at room temperature, then incubated with primary antibodies in 2.5% low fat milk in TBST at 4°C overnight, followed by an incubation with horseradish peroxidase conjugated anti-rabbit or anti-mouse IgG antibodies (1:15,000 in TBST-BSA 2.5% for 1 hour at room temperature). Samples loadings in the different Western blots were controlled with polyclonal antiactin antibodies (1:500). The Western blots were revealed by chemiluminescence using the Super Signal kit (Pierce, Rockford, IL).

**Tissue Samples.** Tumor and matched normal tissue samples were obtained from patients with ccRCC who underwent partial or total nephrectomy from 2002 to 2005. The human Ethics Committee of Rennes University Medical School and Hospital approved this prospective study and all patients signed informed consents. Upon collection, tissue samples were immediately frozen in liquid nitrogen and stored at -80°C in the Centre de Ressources Biologiques (CRB, Rennes, France) and formalin-fixed and paraffin-embedded. In order to determine the VHL gene mutations in the collected samples, denaturing high-performance liquid chromatography (DHPLC) was carried out on a WAVE Nucleic Acid Fragment Analysis system (Transgenomic, Glasgow, UK) with a DNAsep column (Patard et al., Eur. Urol., 2009, 56(5): 794-795). Aberrant peaks were further analyzed by direct sequencing using standard procedures. All mutations were confirmed in a second PCR and sequencing reaction. Multiplex Ligation-dependent Probe Amplification (MLPA), as previously described (Patard, 2009), allowed VHL deletion analysis.

**Cell Culture.** Human cells (Hela, 786-0, RCC4) were maintained in DMEM medium supplemented with 10 % fetal bovine serum and antibiotics and were grown at 37°C in a humidified 5% CO₂ atmosphere. The two primary cell lines (R-180 and R-305) were cultured in RPMI-1640 (Invitrogen, Carlsbad, CA) supplemented with 10% fetal calf serum (Sigma-Aldrich, USA), 1 % penicillin / streptomycin (Sigma-Aldrich, USA) and 10 mM HEPES buffer 1M. HeLa cells were transfected using JetPRIME™ as recommended by the manufacturer (PolyPlus, Ozyme). To overexpress VHL and cells, the Applicants respectively used pCMV-VHL213 and pCMV-VHL172 and pCMV-VHL160 vectors produced in Applicants' laboratory.

**Immunohistochemistry.** Five-µm sections of formalin-fixed paraffin-embedded tissues were transferred to glass slides before immunohistochemical analysis. The samples were incubated in TBST in the presence of 5% BSA. The reactivity against antibodies, and VHL (JD-1956, dilution 1:100) was revealed with the biotin-streptavidin detection system (Dako, Glostrup, Denmark) using diaminobenzidine as the chromogen (Sigma-Aldrich, France). The analyses were performed using a Leica™ DMRXA microscope equipped with a CoolSnapsHQ camera (Photometrics™).

Leica software (Confocal Software 3D) was used for microscope piloting and image acquisition before image analysis by ImageJ (National Institute of Health).

**Antibodies Preparation.** Six mice were immunized with the recombinant protein VHL172(His)₆. After three months, blood was collected for each mice and the immunoreactive capacity of the different blood sample was tested. Mouse N° 28 was sacrificed and the splenocytes were collected. The cell fusion was carried out with immortalized cells. A first selection of fused cells was performed using an ELISA test. The test was performed with the recombinant protein VHL213(His)₆, VHL172(His)₆ and Aurora-(His)₆ in order to discriminate the cells producing antibodies against the histidine-Tag. Among the cells tested, thirty samples were selected and analyzed further.

The 30 clones were analyzed by dot blot analysis. Twenty µg of each recombinant proteins, VHL213(His)₆, VHL172(His)₆ and Aurora-A, were loaded on a polyacrylamide gel and transferred onto a nitrocellulose membrane. Western blots were realized with each clone. Among the clones tested, the Applicants observed 25 clones, which were immunoreactive against the VHL proteins. The positive clones were then tested for their immuoreactivity onto protein extracts from cells expressing exogenous VHL proteins.

### Results

**Identification of the two variants of VHL in different cell lines.** The *vhl* gene encoded two mRNA variants, variant 1 (V1) composed of the three exons, 1, 2 and 3, and variant 2 (V2) composed of the exons 1 and 3. A real time PCR was performed on total RNA extracted from different human cell lines, in order to detect the presence of the two variants in these cell lines (see results presented on Figure 2). As observed on Figure 2, two bands were detected at the molecular size of 308bp for variant 1 and 185bp for variant 2 in all cells tested. However the proportion of the two variants varied from one cell line to another (V2/V1). The larger form is widely detected in the Hela cells and the R305 kidney cells compared to the smaller form (V2). Interestingly, the endothelia cells (HUVEC) and the breast cancer cells (MCF7) showed bands corresponding to the variant 1 and the variant 2 but the ratio V2/V1 was increased in these cells.

**Immunodetection of the three VHL proteins.** As most of the antibodies that have been developed so far to recognize the VHL proteins are poorly specific and inefficient at recognizing the endogenous proteins, in particular pVHL172, the Applicants designed and developed a new monoclonal antibody against human VHL. The Applicants electrophorezed and electro-blotted the three recombinants proteins pVHL213, pVHL172 and pVHL160 that were His- and T7-tagged. The amount of loaded proteins was controlled by silver staining (Figure 3A). The anti-T7 antibody revealed the presence of the three proteins. The membrane was then immunoblotted with the different antibodies against VHL (Figure 3B: left panel: the polyclonal antibody VHL-6030, middle panel the commercial antibody (Sc), right panel the monoclonal antibody VHL-1956). All the antibodies were able to immuno-detect the highest form of pVHL (VHL213). The two forms VHL172 and VHL160 were differentially immuno-revealed by the antibodies tested. The antibodies VHL-6030 and VHL-1956 mainly detected the pVHL172 while the commercial antibody did not present a detectable signal in the immune-reaction. In a very interesting way, the VHL-1956 antibody recognized efficiently the three proteins (Figure 3B) without aspecific bands as observed with the commercial antibody.

The next question was to determine whether the *in cellulo* expressed proteins were detected by the antibody VHL-1956. The Hela cells were transfected with plasmids expressing the different forms of pVHL (Figure 3C). The constructs were tagged with a Flag, as shown on that figure, the three proteins were expressed and immunodetected in Hela cells (Figure 3C, left panel). The same extracts were immunoblotted with the antibodies against VHL (see: Material and Methods). All the three antibodies against VHL detected the pVHL213 and pVHL172 proteins. In contrast, the pVHL160 was only detected using the commercial antibody (Sc) and the monoclonal VHL antibody (VHL-1956). However, it should be noted that no aspecific bands were observable on the membrane immunoblotted with the antibody VHL-1956, while aspecific bands were observed on the immunoblot performed with the commercial antibody (Figure 3C right panel).

The recognition of the recombinant pVHL protein and expressed proteins in cells led the Applicants to investigate the ability of the antibody VHL-1956 to recognize the endogenous expressed proteins. They selected different cell lines (Hela and kidney cells) and performed Western blot with the three VHL antibodies (Figure 4). The amount of proteins loaded on the gel was controlled by tubulin detection. The Western blot performed with the commercial antibody strongly revealed only one molecular size of 23 kDa in all cell lines (Figure 4 upper panel). Two smaller bands were detected in the R-305 and RCC4+ cells which did not fit with the theoretical molecular weight of one of the lower forms of pVHL proteins (Figure 4, upper panel). The two antibodies VHL-6030 and VHL-1956 recognized bands that migrated at the estimated molecular weight of the pVHL proteins. Among these two antibodies, the VHL-6030 antibody detected very slightly the bands (pVHL213*, pVHL172** and pVHL160***), in most of the cell extracts, the protein pVHL172 was not detected by the VHL-6030 antibody (Figure 4 middle panel). In contrast the antibody VHL-1956 strongly detected the three bands (pVHL213*, pVHL172** and pVHL160***) in cell extracts as Hela, RCC4+ and HEK-293T. As expected no bands were detected in the 786-0 cells for which a stop codon is observed at the beginning of the mRNA sequence (Figure 4, lower panel).

All the results contribute to the high capacity of the VHL-1956 antibody to recognize the recombinant proteins, *in vivo* expressed different forms of pVHL and also the endogenous proteins.

**Characterization of the Specificity of the Antibody.** As the antibody VHL-1956 distinguished the native and non-native proteins, the Applicants wondered whether these bands detected with the antibody strictly corresponded to the VHL proteins. The Applicants performed a set of different experiments to improve the specificity of this antibody. In a first experiment, they pre-incubated the antibody with an excess of recombinant protein pVHL213(His)6. They then performed a Western blot with Hela cell extracts (Figure 5A). They observed that the pre-incubated antibody was no more able to detect the endogenous protein (left lane) compared to the Hela cell extract treated with the non-blocked antibody.

In a second round of experiments, the Applicants have targeted the mRNA encoding VHL proteins by a siRNA strategy. They designed two different siRNAs that targeted the three pVHL proteins (siRNA VHL-213) or only the pVHL172 protein (siRNAVHL-172-variant 2). Figure 5B shows the Western blot performed on Hela cell extracts that were transfected with the two distinct siRNAs. The three immunoreactive bands were detected in the Hela cells (Figure 5B left lane). As the siRNAVHL was transfected in Hela cells, the signals corresponding to the three bands strongly decreased (Figure 5B right lane). The transfection of the Hela cells with the siRNAVHL-172 induced a specific decrease of the band corresponding to the pVHL172 when the cell extract (*) was analyzed by Western blot. Another kind of experiment was carried out to confirm the specific recognition of the antibody (Figure 5 C). An siRNAassay was performed with increasing amount of siRNAVHL-172 which emphasized the disappearance of the immune-detected band corresponding to the pVHL172. As shown on the figure, the siRNA scramble has no effect on the detection of the three-immunreactive bands.

**Identification of the VHL protein in tumor tissues extracts.** The above results confirmed that the antibody recognized the three forms of pVHL whether exogenous or endogenous in cells. The antibody allowed the detection of the three pVHL proteins in cells. The Applicants finally tested the antibody on tumor tissue extracts in order to examine the putative expression of the proteins in a patient. Tumor tissues were selected from a CRB-kidney library and treated in order to realize Western blot reactions (Figure 6). The analysis was performed in parallel with a Hela cell extract. Immunoblots revealed bands, which migrated at molecular weight, closed to the proteins detected in the Hela cells. Even though the signal was not as strong as in a cell extract, the Applicants observed that signals corresponding to the different bands pVHL213*, pVHL172** and pVHL160*** were detectable in the tissues. The amount of each of the proteins varied from one patient to another, which can suggest a relationship between the different pVHL isoforms and the disease. For example, the Applicants observed en equivalent ratio between the three forms (*,**,***) in the tumor extract from patient 9 while this ratio was balancing out in favor of the lowest form for patient 3.

**Identification of the antigenic site.** Immunoblots were performed using the VHL-1956 antibody on a peptide map membrane, which carried peptides of 20-mers that recovered the entire VHL sequences. The blot revealed immunoreactive dots onto the peptide -5, -10 and -15 lane 3. The antigenic amino acids were then determined and the Applicants identified the sequence "AGPR" as the immunogene sequence. Moreover the isotype of the antibody was IgG2b-κ.

**Immunostaining of Cell Lines and of Biological Tissues.** The immunostaining of different cell lines (786-0 pVHL213, panel **(b)** and 786-0 pVHL172 **(c)** of Figure 7) revealed a positive labelling of the cells expressing the different forms of pVHL while no labelling was detected in the 786-0 cell line (panel **(a)** of Figure 7). Labelling with also observed in human kidney tissue (panel **(d)** of Figure 7)

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

## Claims

1. A hybridoma cell line deposited at the CNCM on April 18, 2014 under Accession Number CNCM 1-4857.

2. A monoclonal antibody secreted by the hybridoma cell line according to claim 1, or a biologically active fragment thereof, that specifically recognizes pVHL172, pVHL213 and pVHL160, the three isoforms of human pVHL.

3. The monoclonal antibody, or biologically active fragment thereof, according to claim 2, wherein the monoclonal antibody, or biologically active fragment thereof, specifically recognizes a region of human pVHL172 in the interdomain between the acidic domain and the β-domain of human pVHL172, wherein said region of human pVHL172 comprises, or consists of, the sequence set forth in SEQ ID NO: 4 (EAGRPRPVL), or comprises or consists of, the sequence set forth in SEQ ID NO: 5 (AGRPR).

4. The monoclonal antibody, or biologically active fragment thereof, according to any one of claims 2-3, wherein the monoclonal antibody is humanized, de-immunized or chimeric.

5. The monoclonal antibody, or biologically active fragment thereof according to claim 4, wherein the humanized, de-immunized or chimeric monoclonal antibody comprises the six complementary determining regions (CDRs) of the secreted monoclonal antibody according to claim 2.

6. A conjugate comprising a monoclonal antibody, or biologically active fragment thereof, according to any one of claims 2-5, wherein the monoclonal antibody, or biologically active fragment thereof, is attached to a detectable moiety.

7. A kit for detecting the presence of human pVHL in a biological sample or for quantifying the expression level of human pVHL in a biological sample or for isolating human pVHL from a biological sample, comprising a monoclonal antibody, or a biologically active fragment thereof, according to any one of claims 2-5 or a conjugate according to claim 6.

8. The kit according to claim 7 further comprising a monoclonal antibody that specifically recognizes human pVHL213 and/or pVHL160, wherein the monoclonal antibody that specifically recognizes human pVHL213 and/or pVHL160 is optionally attached to a detectable moiety.

9. The kit according to claim 7 or claim 8 further comprising an anti-human IgG optionally attached to a detectable moiety.

10. The kit according to any one of claims 7 to 9, wherein the monoclonal antibody, biologically active fragment thereof or conjugate is immobilized to a solid support.

11. A method for detecting the presence of human pVHL in a biological sample or for quantifying the expression level of human pVHL in a biological sample or for isolating human pVHL from a biological sample, the method comprising a step of contacting the biological sample with a monoclonal antibody, or a biologically active fragment thereof, according to any one of claims 2-5 or with a conjugate according to claim 6.

12. The method according to claim 11, wherein the step of contacting the biological sample is performed for a time and under conditions allowing a complex to form between human pVHL present in the biological sample and the monoclonal antibody, or biologically active fragment thereof, or the conjugate.

13. The method according to claim 12 further comprising a step of detecting the complex formed or a step of quantifying the complex formed.

14. The method according to claim 12, wherein the monoclonal antibody, or biologically active fragment thereof, is attached to a solid support and the method further comprises a step of releasing the pVHL protein from the complex.

15. The kit according to any one of claims 7-10 or the method according to any of claims 11-14, wherein human pVHL is human pVHL172, human pVHL213, pVHL160 or any combination thereof.

## Patentansprüche

1. Hybridomzelllinie, hinterlegt bei der CNCM am 18. April 2014 unter der Zugangsnummer CNCM I-4857.

2. Monoklonaler Antikörper, der sekretiert wird durch die Hybridomzelllinie nach Anspruch 1, oder ein biologisch aktives Fragment davon, das spezifisch pVHL172, pVHL213 und pVHL160, die drei Isoformen von humanem pVHL, erkennt.

3. Monoklonaler Antikörper oder ein biologisch aktives Fragment davon nach Anspruch 2, worin der monoklonale Antikörper oder das biologisch aktive Fragment davon spezifisch eine Region von humanem pVHL172 in der Interdömane zwischen der sauren Domäne und der β-Domäne von humanem pVHL172 erkennt, worin die Region von humanem pVHL172 die Sequenz, die in SEQ ID NO:4 (EAGRPRPVL) angegeben ist, umfasst oder daraus besteht, oder die Sequenz, die in SEQ ID NO:5 (AGRPR) angegeben ist, umfasst oder daraus besteht.

4. Monoklonaler Antikörper oder biologisch aktives Fragment davon nach einem der Ansprüche 2-3, worin der monoklonale Antikörper humanisiert, deimmunisiert oder chimer ist.

5. Monoklonaler Antikörper oder biologisch aktives Fragment davon nach Anspruch 4, worin der humanisierte, deimmunisierte oder chimere monoklonale Antikörper die sechs komplementaritätsbestimmenden Regionen (Complementary Determining Regions (CDR)) des sekretierten monoklonalen Antikörpers nach Anspruch 2 umfasst.

6. Konjugat, umfassend einen monoklonalen Antikörper oder ein biologisch aktives Fragment davon nach einem der Ansprüche 2-5, worin der monoklonale Antikörper oder das biologisch aktive Fragment davon an einen nachweisbaren Rest gebunden ist.

7. Kit zum Nachweisen des Vorliegens von humanem pVHL in einer biologischen Probe oder zum Quantifizieren des Expressionsgrads von humanem pVHL in einer biologischen Probe oder zum Isolieren von humanem pVHL aus einer biologischen Probe, umfassend einen monoklonalen Antikörper, oder ein biologisch aktives Fragment davon nach einem der Ansprüche 2-5 oder ein Konjugat nach Anspruch 6.

8. Kit nach Anspruch 7, weiterhin umfassend einen monoklonalen Antikörper, der spezifisch humanes pVHL213 und/oder pVHL160 erkennt, worin der monoklonale Antikörper, der spezifisch humanes pVHL213 und/oder pVHL160 erkennt, optional an einen nachweisbaren Rest gebunden ist.

9. Kit nach Anspruch 7 oder Anspruch 8, umfassend ein Anti-Human-IgG, optional gebunden an einen nachweisbaren Rest.

10. Kit nach einem der Ansprüche 7 bis 9, worin der monoklonale Antikörper, das biologisch aktive Fragment davon oder das Konjugat an einem festen Träger immobilisiert ist.

11. Verfahren zum Nachweisen des Vorliegens von humanem pVHL in einer biologischen Probe oder zum Quantifizieren des Expressionsgrads von humanem pVHL in einer biologischen Probe oder zum Isolieren von humanem pVHL aus einer biologischen Probe, wobei das Verfahren einen Schritt des In-Kontakt-Bringens der biologischen Probe mit einem monoklonalen Antikörper oder einem biologisch aktiven Fragments davon nach einem der Ansprüche 2-5 oder mit einem Konjugat nach Anspruch 6 umfasst.

12. Verfahren nach Anspruch 11, worin der Schritt des In-Kontakt-Bringens der biologischen Probe durchgeführt wird für eine Zeitdauer und unter Bedingungen, die es erlauben, dass sich ein Komplex zwischen humanem pVHL, das in der biologischen Probe vorliegt, und dem monoklonalen Antikörper oder biologisch aktiven Fragment davon oder dem Konjugat bildet.

13. Verfahren nach Anspruch 12, weiterhin umfassend einen Schritt des Nachweisens des gebildeten Komplexes oder einen Schritt zum Quantifizieren des gebildeten Komplexes.

14. Verfahren nach Anspruch 12, worin der monoklonale Antikörper oder das biologisch aktive Fragment davon an einen festen Träger gebunden wird und das Verfahren weiterhin einen Schritt des Freisetzens des pVHL-Proteins von dem Komplex umfasst.

15. Kit nach einem der Ansprüche 7-10 oder das Verfahren nach einem der Ansprüche 11-14, worin das humane pVHL humanes pVHL172, humanes pVHL213, pVHL160 oder eine beliebige Kombination davon ist.

## Revendications

1. Lignée cellulaire d'hybridome déposée à la CNCM le 18 avril 2014 sous le numéro d'accession CNCM I-4857.

2. Anticorps monoclonal sécrété par la lignée cellulaire d'hybridome selon la revendication 1, ou l'un de ses fragments biologiquement actifs, qui reconnaît spécifiquement la pVHL172, la pVHL213 et la pVHL160, les trois isoformes de la pVHL humaine.

3. Anticorps monoclonal, ou l'un de ses fragments biologiquement actifs, selon la revendication 2, dans lequel l'anticorps monoclonal, ou l'un de ses fragments biologiquement actifs, reconnaît spécifiquement une région de la pVHL172 humaine dans l'interdomaine entre le domaine acide et le domaine β de la pVHL172, dans lequel ladite région de la pVHL172 comprend, ou est constituée de, la séquence représentée par SEQ ID NO: 4 (EAGRPRPVL), ou comprend ou est constituée de la séquence représentée par SEQ ID NO: 5 (AGRPR).

4. Anticorps monoclonal, ou l'un de ses fragments biologiquement actifs, selon l'une quelconque des revendications 2 à 3, dans lequel l'anticorps monoclonal est humanisé, désimmunisé ou chimérique.

5. Anticorps monoclonal, ou l'un de ses fragments biologiquement actifs selon la revendication 4, dans lequel l'anticorps monoclonal humanisé, désimmunisé ou chimérique comprend les six régions déterminant la complémentarité (CDR) de l'anticorps monoclonal sécrété selon la revendication 2.

6. Conjugué comprenant un anticorps monoclonal, ou l'un de ses fragments biologiquement actifs, selon l'une quelconque des revendications 2 à 5, dans lequel l'anticorps monoclonal, ou l'un de ses fragments biologiquement actifs, est fixé à une fraction détectable.

7. Kit pour détecter la présence de la pVHL humaine dans un échantillon biologique ou pour quantifier le taux d'expression de la pVHL humaine dans un échantillon biologique ou pour isoler la pVHL humaine à partir d'un échantillon biologique, comprenant un anticorps monoclonal, ou l'un de ses fragments biologiquement actifs selon l'une quelconque des revendications 2 à 5 ou un conjugué selon la revendication 6.

8. Kit selon la revendication 7 comprenant en outre un anticorps monoclonal qui reconnaît spécifiquement la pVHL213 et/ou la pVHL160 humaine, dans lequel l'anticorps monoclonal qui reconnaît spécifiquement la pVHL213 et/ou la pVHL160 humaine est éventuellement fixé à une fraction détectable.

9. Kit selon la revendication 7 ou la revendication 8 comprenant en outre une IgG anti-humaine éventuellement fixée à une fraction détectable.

10. Kit selon l'une quelconque des revendications 7 à 9, dans lequel l'anticorps monoclonal, l'un de ses fragments biologiquement actifs ou le conjugué est immobilisé sur un support solide.

11. Procédé pour détecter la présence de la pVHL humaine dans un échantillon biologique ou pour quantifier le taux d'expression de la pVHL humaine dans un échantillon biologique ou pour isoler la pVHL humaine à partir d'un échantillon biologique, le procédé comprenant une étape de mise en contact de l'échantillon biologique avec un anticorps monoclonal, ou l'un de ses fragments biologiquement actifs, selon l'une quelconque des revendications 2 à 5 ou avec un conjugué selon la revendication 6.

12. Procédé selon la revendication 11, dans lequel l'étape de mise en contact de l'échantillon biologique est effectuée pendant un temps et dans des conditions permettant à un complexe de se former entre la pVHL humaine présente dans l'échantillon biologique et l'anticorps monoclonal, ou l'un de ses fragments biologiquement actifs, ou le conjugué.

13. Procédé selon la revendication 12 comprenant en outre une étape de détection du complexe formé ou une étape de quantification du complexe formé.

14. Procédé selon la revendication 12, dans lequel l'anticorps monoclonal, ou l'un de ses fragments biologiquement actifs, est fixé à un support solide et le procédé comprend en outre une étape de libération de la protéine pVHL à partir du complexe.

15. Kit selon l'une quelconque des revendications 7 à 10 ou procédé selon l'une quelconque des revendications 11 à 14, dans lequel la pVHL humaine est la pVHL172 humaine, la pVHL213 humaine, la pVHL160 ou l'une quelconque de leurs combinaisons.
